# EUROPEAN PATENT APPLICATION

(11) **EP 4 176 883 A1**
(43) Date of publication of application: **10.05.2023**
(21) Application number: 21817195.7
(22) Date of filing: 03.06.2021
(51) Int. Cl.: A61K 31/536, A61K 31/138, A61K 31/15, A61K 31/4525, A61K 31/135, A61K 31/505, A61K 31/381, A61K 31/55, A61K 31/137, A61K 45/06

(54) **PHARMACEUTICAL COMPOSITION FOR PREVENTING OR TREATING CANCER CONTAINING ANTIVIRAL AGENT AND ANTIDEPRESSANT AS ACTIVE INGREDIENTS**

(30) Priority: 05.06.2020 KR 20200068221
(71) Applicant: FRONTBIO INC., Chuncheon-si, Gangwon-do 24232 (KR)
(72) Inventor: PARK, Soo Hyun, Chuncheon-si Gangwon-do 24267 (KR); JANG, Young Su, Chuncheon-si Gangwon-do 24453 (KR); KIM, Set Byeol, Chuncheon-si Gangwon-do 24322 (KR); KIM, Jeong Hoon, Chuncheon-si Gangwon-do 24277 (KR)
(74) Representative: Vossius & Partner Patentanwälte Rechtsanwälte mbB
(86) International application number: PCT/KR2021/006915
(87) International publication number: WO 2021/246797

(57) **Abstract**

The present invention relates to a pharmaceutical composition for preventing or treating cancer comprising an antiviral agent and an antidepressant as an active ingredient and to which a biguanide-based compound can be added, and more specifically, efavirenz, etravirine, rilpivirine and lopinavir, as antiviral agents, and fluoxetine, fluvoxamine, paroxetine, sertraline, duloxetine, amitriptyline, clomipramine, nortriptyline, desipramine, amoxapine, maprotiline, trimipramine, protriptyline and melitracen, as antidepressants, in complex, mixture, or combination administration, or efavirenz and fluoxetine with metformin, a biguanide compound, in complex, mixture, or combination administration, shows the significant synergistic anti-cancer activity than when each was administered alone, and so, finally the antiviral agent and antidepressant according to the present invention can be effectively used as an active ingredient in a composition for preventing or treating cancer.

## Description

### [Technical Field]

The present invention relates to a pharmaceutical composition for preventing or treating cancer comprising an antiviral agent and an antidepressant as active ingredients, and more specifically a pharmaceutical composition for preventing or treating comprising a first component including an antiviral agent; and a second component including an antidepressant as active ingredients by a complex, mixed or combined preparation. The present invention also relates to a pharmaceutical composition for preventing or treating cancer comprising the said first component; the said second component; and a biguanide-based compound or a pharmaceutically acceptable salt thereof to a third component.

### [Background Art]

Cancer is a disease caused by the uncontrolled growth of abnormal cells that may spread in contact with tissues or other parts of the body. Cancer cells may form solid tumors in which the cancer cells cluster together or may exist as dispersed cells as in leukemia.

Normal cells differentiate until they mature and then replace damaged or dead cells as needed, but cancer cells constantly differentiate and eventually they push out nearby cells and spread to other parts, which is called malignancy. Malignant tumor cells metastasize to other parts of the body through the bloodstream or lymphatic system, where they proliferate and form new tumors.

Despite of the development of various treatment methods, cancer still seriously threatens human health worldwide. Current major cancer treatment methods include surgery, radiation therapy, hormone therapy, and chemotherapy, and among these, chemotherapy is a method for treating cancer directly or relieving symptoms using one or more anticancer drugs.

Traditional chemotherapeutic agents exhibit cytotoxicity to cancer cells in a way of interfering the division and metabolism of cancer cells or suppressing the biosynthesis of nucleic acids or proteins. However, these chemotherapeutic agents have problems of that cancer cells have resistance to those anticancer drugs and causing serious side effects of that an anticancer drug exhibits toxicity to normal tissues. Specifically, substances used as existing anticancer drugs affect not only cancer cells but also are toxic to normal cells and thus cause various side effects in many cases. Therefore, there is a need for an anticancer drug that is not toxic to normal cells yet exhibits excellent selective toxicity only to cancer cells and exhibits excellent anticancer activity.

Antiviral agents are drugs to treat infectious diseases caused by viruses in a way of weakening or eliminating the virus actions invading human bodies, so the antiviral agents have an action of inhibiting the proliferation of viruses. According to the action mechanism of the antiviral agent, it can be classified into virus attachment, penetration inhibitors, and virus growth inhibitors. Most antiviral agents exhibit antiviral activity by inhibiting several types of enzymes necessary for the virus growth.

Human Immunodeficiency Virus (HIV), particularly strains known as HIV type-1 (HIV-1) and type-2 (HIV-2), is etiologically associated with the immunosuppressive disease known as Acquired Immunodeficiency Syndrome(AIDS). Since HIV is a retrovirus, the HIV replication cycle requires transcription of the viral RNA genome into DNA via reverse transcriptase. Therefore, compounds that inhibit the enzymatic action of HIV reverse transcriptase are being developed as HIV antiviral agents.

Compounds that inhibit the enzyme action of HIV reverse transcriptase include Nucleoside Reverse Transcriptase Inhibitor (NRTI) compounds and Non-nucleoside Reverse Transcriptase Inhibitor (NNRTI) compounds. NRTIs are mainly taken as precursors, and then enter the host cell and become phosphorylated and converted into nucleotides to become active as drugs. Since NRTIs commonly lack the 3-OH (hydroxyl group) of the sugar molecule of the nucleoside, they intervene in the DNA strand during viral DNA synthesis and block the 3'-5' phosphate ester bond with the next nucleotide in the DNA chain, and finally by doing so, DNA synthesis is terminated. FDA-approved NRTIs include zidovudine, didanosine, zalcitabine, stavudine, lamivudine, abacavir, tenofovir disoproxil fumarate), emtricitabine, and tenofovir alafenamide fumarate. Unlike NRTI, NNRTI does not act as a viral DNA synthesis terminator, but directly binds to the hydrophobic pocket near the active site of reverse transcriptase and changes the enzyme structure, thereby inhibiting enzyme activity. FDA-approved NNRTIs include efavirenz, etravirine, nevirapine, doravirine, rilpivirine, and delavirdine.

Protease inhibitors (PIs) are compounds to inhibit the enzyme that converts the precursor proteins of Gag and Gag-Pol contained in non-infectious virions into infectious mature viruses. Although it was expected that the enzyme plays a very important role in virus propagation and would be very low occurrence of resistance due to its very small size at 11 kDa, however resistance mutations occur with a very high frequency, and thus it is currently mainly used only for `cocktail therapy' in which three anti-HIV drugs are administered. PIs include saquinavir, ritonavir, indinavir, nelfinavir, amprenavir, lopinavir, atazanavir, fosamprenavir, tipranavir, and darunavir.

In recent years, the anticancer effect of these antiviral agents has been studied. For example, efavirenz has been reported to have anticancer effects in glioblastoma, pancreatic cancer, and ovarian cancer, etravirine has been reported to have anticancer effects in ovarian cancer, nevirapine has been reported to have cancer cell metastasis inhibitory effects in liver cancer and thyroid cancer, and the combination of nelfinavir, lopinavir and vincristine has an effect of reducing the viability of oral squamous cell carcinoma cells.

In addition, antidepressants are drugs to improve depressive symptoms by controlling the imbalance of neurotransmitters (serotonin, norepinephrine, dopamine) related to depression, and can be classified into tricyclic antidepressants (TCA), monoamine oxidase inhibitors (MAOIs), selective serotonin reuptake inhibitors (SSRIs), and serotonin-norepinephrine reuptake inhibitors (SNRIs).

Tricyclic antidepressants are substances to improve depressive symptoms by blocking the reuptake of serotonin and norepinephrine at nerve cell terminals and increasing the concentration of neurotransmitters in the synapse, and include nortriptyline, imipramine, and amoxapine.

Selective serotonin reuptake inhibitors are substances that selectively block serotonin reuptake at nerve cell terminals to increase the concentration of serotonin in the synapse, thereby increasing the activity of serotonin and alleviating depressive symptoms, and include fluoxetine, fluvoxamine, paroxetine, sertraline, escitalopram, and vortioxetine, which are used for depression, obsessive-compulsive disorder, and premenstrual dysphoric disorder. Compared to tricyclic antidepressants, it is superior in terms of side effects and safety, and is most often used as an antidepressant.

Recently, the anticancer effect of these antidepressants has been reviewed. For example, imipramine has been reported to have an anticancer effect in small cell lung cancer, and fluoxetine has been reported to have an anticancer effect in hepatocellular carcinoma and non-small cell lung cancer.

Metformin, phenformin, buformin, and biguanide are all biguanide-based drugs, and are still widely used as medications for type 2 diabetes, which inhibit the production of glucose in the liver and promote the use of glucose in peripheral blood vessels. Metformin, phenformin, buformin, or biguanide activates AMPK (AMP-activated protein kinase), a key enzyme in metabolic regulation, to inhibit the synthesis of protein, fat lipid, and glycogen and promote the degradation thereof, and inhibits the production of insulin, IGF1, leptin, and adiponectin. Meanwhile, activated AMPK suppresses cell regeneration, and thus inhibits metabolism of cancer cells and inhibits cell division. AMPK activation suppresses the proliferation of cancer cells by directly inhibiting mTOR (mammalian target of rapamycin) and eventually inhibiting protein synthesis. In particular, it has been reported that metformin suppresses the growth of cancer cells by inhibiting the expression of angiogenesis promoters. Due to this anticancer mechanism, metformin and phenformin have been used in clinical trials of various kinds of cancers alone or in combination with other anticancer drugs, but the therapeutic effect varies, and metformin and phenformin have not yet been approved as anticancer drugs because of several problems.

Accordingly, the present inventors have studied to develop a combination of substances having a superior anticancer effect, as a result, revealed the fact that a combination of an antiviral agent, antidepressant and biguanide-based compound shows a remarkable synergism in the anticancer effect, and thus applied for a new complex, mixed, or combined anticancer drug.

### [Prior Art List]

### [Patent Literature]

US 2014-0113930 A1 (2014.4.24)

### [Non Patent Literature]

Lopez, J.; Tait, S.W.G. Mitochondrial apoptosis: Killing cancer using the enemy within. Br. J. Cancer 2015, 112, 957-962.
Yip, K.W.; Reed, J.C. BCL-2 family proteins and cancer. Oncogene 2008, 77, 6398-6406.
Elkholi, R.; Renault, T.T.; Serasinghe, M.N.; Chipuk, J.E. Putting the pieces together: How is the mitochondrial pathway of apoptosis regulated in cancer and chemotherapy? Cancer Metab. 2014, 2, 16.
Fiocchetti, M.; Nuzzo, M.T.; Totta, P.; Acconcia, F.; Ascenzi, P.; Marino, M. Neuroglobin, a pro-survival player in estrogen receptor-positive cancer cells. Cell Death Dis. 2014, 5, e1449.
J. Xu, and W. Mao Overview of Research and Development for Anticancer Drugs, Journal of Cancer Therapy, 2016, 7, 762-772.
Faubert B, Vincent EE, Poffenberger MC, Jones RG. The AMPactivated protein kinase (AMPK) and cancer: many faces of a metabolic regulator. Cancer Lett 2015;356:165-70.
Zoncu R, Efeyan A, Sabatini DM. mTOR: from growth signal integration to cancer, diabetes and ageing. Nat Rev Mol Cell Biol 2011;12(1):21-35.
Eric J. Arts and Daria J. Hazuda. HIV-1 Antiretroviral Drug Therapy. Cold Spring Harb Perspect Med. 2012;2:a007161.
Mingzhi Han, Shuai Wang, Ning Yang, Xu Wang, et al. Therapeutic implications of altered cholesterol homeostasis mediated by loss of CYP46A1 in human glioblastoma. EMBO Mol Med. 2020 Jan 9;12(1):e10924.
Hecht M, Harrer T, et al. Cytotoxic effect of Efavirenz in BxPC-3 pancreatic cancer cells is based on oxidative stress and is synergistic with ionizing radiation. Oncol Lett. 2018 Feb;15(2):1728-1736.
Perna A, Lucariello A, Sellitto C, et al. Different Cell Cycle Modulation in SKOV-3 Ovarian Cancer Cell Line by Anti-HIV Drugs. Oncol Res. 2017 Nov 2;25(9):1617-1624.
Shang H, Zhao J, et al. Nevirapine inhibits migration and invasion in dedifferentiated thyroid cancer cells. Thorac Cancer. 2019 Dec;10(12):2243-2252.
Kim JY, Park YJ, et al. Co-treatment With HIV Protease Inhibitor Nelfinavir Greatly Increases Late-phase Apoptosis of Drug-resistant KBV20C Cancer Cells Independently of P-Glycoprotein Inhibition. Anticancer Res. 2019 Jul;39(7):3757-3765.
Jahchan NS, Dudley JT, Mazur PK, et al. A drug repositioning approach identifies tricyclic antidepressants as inhibitors of small cell lung cancer and other neuroendocrine tumors. Cancer Discov. 2013 Dec;3(12):1364-77. doi: 10.1158/2159-8290.CD-13-0183.
Hsu LC, Tu HF, et al. Beneficial effect of fluoxetine on anti-tumor progression on hepatocellular carcinoma and non-small cell lung cancer bearing animal model. Biomed Pharmacother. 2020 Mar 4;126:110054.
Dallaglio K, Bruno A, Cantelmo AR, Esposito AR, Ruggiero L, Orecchioni S, et al. Paradoxic effects of metformin on endothelial cells and angiogenesis. Carcinogenesis 2014;35:1055-66.

### [Detailed Description]

### [Technical Problem]

Substances used as existing anticancer drugs affect not only cancer cells but also are toxic to normal cells, for example, rapidly dividing normal cells such as skin, mucous membranes, and blood cells, and thus the substances cause various side effects in many cases such as hair loss, diarrhea, and leukopenia. Unlike normal cells, the expression of antiapoptotic proteins such as BCL-2 is increased or the expression of proapoptotic proteins such as BAX is suppressed, and apoptosis is often lacked in many cancer cells. In cancer cells, the expression of caspases may be low or mutations in the caspase gene may occur. In some cases, apoptosis may be inhibited as mitochondrial outer membrane permeabilization (MOMP) is inhibited in cancer cells. As described above, since apoptosis does not occur in many cancer cells, there is a problem that the therapeutic effect of many anticancer drugs that induce apoptosis is not obtained. Hence, there is an urgent demand for an anticancer drug that exhibits excellent anticancer activity while not being toxic to normal cells but exhibiting excellent selective toxicity only to cancer cells.

### [Technical Solution to Problem]

The present invention relates to a complex, mixed, or combined pharmaceutical preparation for use in the prevention or treatment of cancer, more specifically, a pharmaceutical composition comprising a first component including an antiviral agent; and a second component including an antidepressant as active ingredient, exhibits a remarkably excellent anticancer effect against cancer cells, and the above problem is solved by providing a pharmaceutical composition comprising the first component and the second component as active ingredients for use in the form of a complex, mixed, or combined preparation.

In addition, the present invention also resolves the above problem by providing a pharmaceutical composition, for preventing or treating cancer, comprising the said first component; the said second component; and a biguanide-based compound or a pharmaceutically acceptable salt thereof to a third component for use in the form of a complex, mixed, or combined preparation.

In one embodiment of the invention, the antiviral agent comprises nonnucleoside reverse transcription (NNRTIs; Non-nucleoside reverse-transcriptase inhibitors) family compounds or derivatives thereof, or pharmaceutically acceptable salts thereof, and the NNRTI family compounds or derivatives thereof may be selected from the group consisting of efavirenz, etravirine, nevirapine, doravirine, rilpivirine, and delavirdine.

In one embodiment of the present invention, the antiviral agent comprises nucleoside reverse transcription (NRTIs; Nucleoside reverse-transcriptase inhibitors) family compounds or derivatives thereof, or pharmaceutically acceptable salts thereof, and the NRTI family compounds or derivatives thereof may be selected from the group consisting of zidovudine, didanosine, zalcitabine, stavudine, lamivudine, abacavir, tenofovir disoproxil fumarate, emtricitabine, tenofovir alafenamide fumarate.

In one embodiment of the present invention, the antiviral agent comprises proteolytic inhibitors (PIs; Protease inhibitors) family compounds or derivatives thereof, or pharmaceutically acceptable salts thereof, such PI family compounds or derivatives thereof may be selected from the group consisting of saquinavir, ritonavir, indinavir, nelfinavir, amprenavir, lopinavir, atazanavir, fosamprenavir, tipranavir and darunavir.

In one embodiment of the present invention, the antiviral agent comprises an integratose inhibitor (INSTI; It may be an Integrase Strand Transfer Inhibitor) family compound or derivative thereof, or a pharmaceutically acceptable salt thereof, and the INSTI family compound or derivative thereof may be dolutegravir and raltegravir.

In one embodiment of the present invention the antiviral agent may be a fusion inhibitor family compound or derivative thereof, or a pharmaceutically acceptable salt thereof, and the fusion inhibitor may be an enfuvirtide.

In one embodiment of the present invention, the antiviral agent may be a CCR 5 (Chemokine (C-C motif) ligand 5) inhibitor family compound or derivative thereof, or a pharmaceutically acceptable salt thereof, wherein the CCR 5 inhibitory compound or derivative thereof is a single agent maraviroc and the compound comic cobishit / elvitegravir / emtricitabine / tenofovir alafenamide (cobicistat / elvitegravir / emtricitabine / Tenofovir alafenamide), cobicistat / Elvitegravir / emtricitabine / tenofovir disoproxil (cobicistat / Elvitegravir / emtricitabine / Tenofovir disoproxil) and abacabir / dogravir / lamivudine / lamivudine.

In one embodiment of the present invention, the antiviral agent may be a flu therapeutic agent, wherein the flu therapeutic agent is an uncoating inhibitor family compound or derivative thereof, or a pharmaceutically acceptable salt thereof, amantadine, rimantadine and neuraminidase inhibitors (NAIs; Neuraminidase inhibitors) family of compounds or derivatives thereof, or pharmaceutically acceptable salts thereof, and may be selected from the group consisting of oseltamivir, zanamivir, peramivir, baloxavir and laninamivir.

In one embodiment of the present invention, the antiviral agent may be a herpes therapeutic agent, wherein the herpes therapeutic agent may be selected from the group consisting of aciclovir, valaciclovir, idoxuridine, vidarabine, penciclovir, famciclovir, trifluridine, cidofovir and foscarnet.

In one embodiment of the present invention, the antiviral agent may be a treatment for hepatitis B, wherein the hepatitis B therapeutic agent may be selected from the group consisting of lamivudine, clevudine, telbivudine, entecavir, adefovir, tenofovir disoproxil, tenofovir alafenamide and besifovir.

In one embodiment of the present invention, the antiviral agent may be a treatment for hepatitis C, wherein the hepatitis C therapeutic agent is a cytokine peginterferon alpha 2a (peginterferon-α-2a) and peginterferon alpha 2b (peginterferon-α-2b), a compound or derivative thereof, or a pharmaceutically acceptable salt thereof, ribavirin, an antiviral agent acting on a viral protein (DAA; Direct-acting antivirals) family compounds or derivatives thereof, or pharmaceutically acceptable salts thereof, may be selected from the group consisting of the single agents boceprevir, dasabuvir, daclatasvir, asunaprevir, sofosbuvir and the complex elbasvir / grazoprevir, glecaprevir / pibrentasvir, ombitasvir / paritaprevir / ritonavir.

In one embodiment of the present invention the antiviral agent may be an immunoenhancer (interferon) family compound or derivative thereof, or a pharmaceutically acceptable salt thereof, and the immunoenhancer may be selected from the group consisting of interferon alfa-2a, interferon alfa-2b and peginterferon-α-2a.

In one embodiment of the present invention the antiviral agent may be an immunoresponse modulator family compound or derivative thereof, or a pharmaceutically acceptable salt thereof, and the immune response modulator may be an imiquimod.

In one embodiment of the invention, the antidepressant comprises tricyclic antidepressants (Tricyclic antidepressants; TCAs), and the tricyclic antidepressant may be selected from the group consisting of amitriptyline, nortriptyline, clomipramine, imipramine, amoxapine, desipramine, maprotiline, trimipramine, protriptyline and melitracen.

In one embodiment of the present invention, the antidepressants may be selective serotonin reuptake inhibitors (SSRIs), and the selective serotonin reuptake inhibitors may be selected from the group consisting of fluoxetine, fluvoxamine, paroxetine, sertraline, escitalopram and vortioxetine.

In one embodiment of the invention, the antidepressant comprises a monoamine oxidase inhibitor (Monoamine oxidase inhibitor; MAOI), and the monoamine oxidase inhibitor may be moclobemide.

In one embodiment of the invention, the antidepressant comprises Serotonin-norepinephrine reuptake inhibitors; SNRIs), and the serotonin norepinephrine reuptake inhibitors may be selected from the group consisting of duloxetine, venlafaxine, desbenlafaxine, milnacipran and the antidepressants bupropion, mirtazapine, trazodone, and tianeptine.

In one embodiment of the invention, the biguanide-based compound may be selected from the group consisting of metformin, phenformin, buformin and biguanide.

In one embodiment of the invention, the first component antiviral; and the second component antidepressant may be combined with the first component 0.0000001 to 10 parts by weight: the second component 1 weight ratio.

In one embodiment of the invention, the first component antiviral; the second component antidepressants; and the third component biguanide based compounds or pharmaceutically acceptable salts thereof are, the first component 0.0000001 to 10 parts by weight; the second component 1 by weight part; and the third component 0.01 to 100000 may be combined in a weight ratio.

In one embodiment of the present invention, the salt may be selected from the group consisting of an inorganic acid such as hydrochloric acid, nitric acid, phosphoric acid, sulfuric acid, hydrobromic acid, hydroglycerides iodilate, nitrite acid or aphosphate, aliphatic mono and dicarboxylate, phenyl-substituted alkanoate, hydroxyalkanoate and alkandioate, aromatic acids, aliphatic and aromatic sulfonic acids, pharmaceutically toxic salts sulfate, pyrosulfate, bisulfate, sulfites, bisulfites, bisulfites, nitrates, phosphates, monohydrogen phosphates, dihydrogen phosphates, methaphosphates, pyrophosphate chloride, bromide, iodide, fluoride, acetate, propionate, decanoate, Caprylate, Acrylate, Pomate, Isobutyrate, Caprate, Heptanoate, Propiolate, Oxalate, Malonate, Succinate, Suberrate, Sebacate, Fumarate, Maliate, Butin-1,4-dioate, hexane-1,6-dioate, benzoate, chlorobenzoate, methylbenzoate, dinitro benzoate, hydroxybenzoate, methoxybenzoate, phthalate, terephthalate, benzensulfonate, toluenesulfonate, chlorobenzenesulfonate, xylenesulfonate, phenylacetate, phenylpropionate, phenylbutyrate, citrate, lactate, hydroxybutyrate, glycolate, malate, tartrate, methanesulfonate, propanesulfonate, naphthalene-1-sulfonate, naphthalene-2-sulfonate or mandelate and metal salts such as sodium, potassium and calcium.

In one embodiment of the present invention, the cancer may be selected from the group consisting of (A) breast cancers including (1) ductal carcinoma including ductal carcinoma in situ (DCIS) (comedocarcinoma, cribriform, papillary, micropapillary), invasive ductal carcinoma (IDC), ductal carcinoma, mucinous (colloidal) carcinoma, papillary carcinoma, metaplastic carcinoma and inflammatory carcinoma; (2) lobular carcinomas, including lobular carcinoma in situ (LCIS) and invasive lobular carcinoma; and (3) Paget's disease of the nipple; (B) cancers of the female reproductive system, including (1) cancers of the cervix, including cervical intraepithelial neoplasia (grade I), cervical intraepithelial neoplasia (grade II), cervical intraepithelial neoplasia (grade III) (squamous cell carcinoma in situ), keratinizing squamous cell carcinoma, nonkeratinizing squamous cell carcinoma, verrucous carcinoma, adenocarcinoma in situ, adenocarcinoma in situ, endometrial type carcinoma, endometrioid adenocarcinoma, clear cell adenocarcinoma, adenoepithelioma, adenoid cystic carcinoma, small cell carcinoma and undifferentiated carcinoma; (2) cancers of the uterine body, including endometrioid carcinoma, adenocarcinoma, adenoacanthoma (adenocarcinoma with squamous metaplasia), adenoepithelioma (mixed adenocarcinoma and squamous cell carcinoma), mucinous adenocarcinoma, serous adenocarcinoma, clear cell adenocarcinoma, squamous cell adenocarcinoma and undifferentiated adenocarcinoma; (3) cancers of the ovary, including serous cystadenoma, serous cystadenoma, mucinous cystadenoma, mucinous cystadenoma, endometrioid tumor, endometrioid adenocarcinoma, clear cell tumor, clear cell cystadenoma and unclassified tumors; (4) cancers of the vagina, including squamous cell carcinoma and adenocarcinoma; and (5) vulvar cancers, including vulvar intraepithelial neoplasia (grade I), vulvar intraepithelial neoplasia (grade II), vulvar intraepithelial neoplasia (grade III) (squamous cell carcinoma in situ); squamous cell carcinoma, verrucous carcinoma, Paget's disease of the vulva, adenocarcinoma (NOS); basal cell carcinoma (NOS) and Bartholin gland carcinoma; (C) cancers of the male reproductive system, including (1) cancer of the penis, including squamous cell carcinoma; (2) cancers of the prostate, including adenocarcinomas, sarcomas, and transitional cell carcinomas of the prostate; and (3) cancers of the testes, including seminoma tumor, non-seminoma tumor, teratomas, embryonic carcinomas, yolk sac tumor and choriocarcinoma; (D) cancers of the heart system, including sarcomas (angiosarcoma, fibrosarcoma, rhabdomyosarcoma, liposarcoma), myxoma, rhabdomyoma, fibroma, lipoma and teratoma; (E) cancers of the respiratory system, including squamous cell carcinoma of the larynx, primary pleural mesothelioma and squamous cell carcinoma of the pharynx; (F) cancers of the lung, including squamous cell carcinoma (epidermoid carcinoma), a variant of squamous cell carcinoma, spindle cell carcinoma, small cell carcinoma, carcinoma of other cells, carcinoma of the intermediate cell type, complex oat cell carcinoma, adenocarcinoma, acinar adenocarcinoma, papillary adenocarcinoma, bronchoalveolar carcinoma, mucin-producing solid carcinoma, giant cell carcinoma, giant cell carcinoma, clear cell carcinoma and sarcoma; (G) cancers of the gastrointestinal tract, including (1) cancers of the ampulla of vater, including primary adenocarcinoma, carcinoid tumor and lymphoma; (2) cancers of the anal canal, including adenocarcinoma, squamous cell carcinoma and melanoma; (3) cancers of the extrahepatic bile duct, including carcinoma in situ, adenocarcinoma, papillary adenocarcinoma, adenocarcinoma, intestinal type, mucinous adenocarcinoma, clear cell adenocarcinoma, signet ring cell carcinoma, adenoepithelioma, squamous cell carcinoma, small cell (oat cell) carcinoma, undifferentiated carcinoma, carcinoma (NOS), sarcoma and carcinoid tumor; (4) cancers of the colon and rectum, including adenocarcinoma in situ, adenocarcinoma, mucinous adenocarcinoma (colloidal type; > 50% mucinous carcinoma), signet ring cell carcinoma (greater than 50% of signet ring cells), squamous cell (epidermoid) carcinoma, adenoepithelioma, small cell (oat cell) carcinoma, undifferentiated carcinoma, carcinoma (NOS), sarcoma, lymphoma and carcinoid tumor; (5) cancers of the esophagus, including squamous cell carcinoma, adenocarcinoma, leiomyosarcoma and lymphoma; (6) cancers of the gallbladder, including adenocarcinoma, adenocarcinoma, bowel type, adenoepithelioma, carcinoma in situ, carcinoma (NOS), clear cell adenocarcinoma, mucinous adenocarcinoma, papillary adenocarcinoma, signet ring cell carcinoma, small cell (oat cell) carcinoma, squamous cell carcinoma and undifferentiated carcinoma; (7) cancers of the lips and oral cavity, including squamous cell carcinoma; (8) cancers of the liver, including liver cancer (hepatocellular carcinoma), cholangiocarcinoma, hepatoblastoma, hemangiosarcoma, hepatocellular adenoma and hemangioma; (9) cancers of the exocrine pancreas, including ductal cell carcinoma, giant cell carcinoma multiforme, giant cell carcinoma, osteoclastoid type, adenocarcinoma, adenoepithelioma, mucinous (colloidal) carcinoma, cystadenoma, acinar cell carcinoma, papillary carcinoma, small cell (oat cell) carcinoma, mixed cell type, carcinoma (NOS), undifferentiated carcinoma, endocrine cell tumor arising from islet cells of Langerhans and carcinoid tumor; (10) cancers of the salivary glands, including acinar (acinar) cell carcinoma, adenoid cystic carcinoma (cylindroma), adenocarcinoma, squamous cell carcinoma, carcinoma in pleomorphic adenoma (malignant mixed tumor), mucoepidermoid carcinoma (well-differentiated or low grade) and mucoepidermoid carcinoma (poorly differentiated or high grade); (11) cancers of the stomach, including adenocarcinoma, papillary adenocarcinoma, tubular adenocarcinoma, mucinous adenocarcinoma, signet ring cell carcinoma, adenoepithelioma, squamous cell carcinoma, small cell carcinoma, undifferentiated carcinoma, lymphoma, sarcoma and carcinoid tumor; and (12) cancers of the small intestine, including adenocarcinoma, lymphoma, carcinoid tumor, Kaposi's sarcoma, leiomyoma, hemangioma, lipoma, neurofibromatosis and fibroma; (H) cancers of the urinary system, including (1) cancers of the kidney, including renal cell carcinoma, carcinoma of the Bellini collecting duct, adenocarcinoma, papillary carcinoma, tubular carcinoma, granular cell tumor, clear cell carcinoma (adenocarcinoma of the kidney), sarcoma of the kidney and nephroblastoma; (2) cancers of the renal pelvis and ureter, including transitional cell carcinoma, papillary transitional cell carcinoma, squamous cell carcinoma and adenocarcinoma; (3) cancers of the urethra, including transitional cell carcinoma, squamous cell carcinoma and adenocarcinoma; and (4) cancers of the bladder, including carcinoma in situ, transitional urothelial cell carcinoma, papillary transitional cell carcinoma, squamous cell carcinoma, adenocarcinoma, and undifferentiated carcinoma; and(I) cancers of muscles, bones and soft tissues, including (1) cancers of the bone, including (a) osteogenesis: osteosarcoma; (b) chondrogenesis: chondrosarcoma and mesenchymal chondrosarcoma; (c) giant cell tumor, malignant; (d) Ewing's sarcoma; (e) vascular tumors: hemangioendothelioma, hemangiopericytoma and hemangiosarcoma; (f) connective tissue tumors: fibrosarcoma, liposarcoma, malignant mesenchymoma and undifferentiated sarcoma; and (g) other tumors: chordoma and adamantinoma of the long bones; (2) cancers of soft tissues, including alveolar soft part sarcoma, angiosarcoma, epithelioid sarcoma, extraosseous chondrosarcoma, fibrosarcoma, leiomyosarcoma, liposarcoma, malignant fibrous histiocytoma, malignant hemangiopericytoma, malignant mesenchymoma, malignant Schwannoma, rhabdomyosarcoma, synovial sarcoma and sarcoma (NOS); (3) cancers of the nervous system, including cancers of the skull (osteoma, hemangioma, granuloma, xanthoma, and osteitis deformans), cancers of the meninges (meningioma, meningiosarcoma, and gliomatosis), cancers of the brain (astrocytoma, meduloblastoma, glioma, ependymal glioma, germinoma (pineal tumor), glioblastoma multiforme, oligodendrocytoma, schwannoma, retinoblastoma, and congenital tumor), and cancers of the spinal cord (neurofibromatosis, meningioma, glioma, sarcoma); (4) hematologic malignancies, including myeloid leukemia (acute and chronic), acute lymphoblastic leukemia, chronic lymphocytic leukemia, myeloproliferative disease, multiple myeloma; myelodysplastic syndrome, Hodgkin's disease and non-Hodgkin's lymphoma (malignant lymphoma); (5) cancers of the endocrine system, including (a) cancers of the thyroid gland, including papillary carcinoma (including those of the follicular region), follicular carcinoma, medullary carcinoma and undifferentiated (anaplastic) carcinoma; and (b) neuroblastomas, including sympathicogonioma, sympathoblastoma, malignant ganglioneuroma, ganglioneuroblastoma and ganglioneuroma; (6) cancers of the skin, including squamous cell carcinoma, spindle cell squamous cell carcinoma, basal cell carcinoma, adenocarcinoma arising from sweat glands or sebaceous glands, and malignant melanoma; and (7) cancers of the eye, including (a) cancer of the conjunctiva, including carcinoma of the conjunctiva; (b) cancers of the eyelid, including basal cell carcinoma, squamous cell carcinoma, melanoma of the eyelid and sebaceous cell carcinoma; (c) cancers of the lacrimal gland, including adenocarcinoma, adenoid cystic carcinoma, carcinoma in pleomorphic adenoma, mucoepidermoid carcinoma and squamous cell carcinoma; (d) cancers of the uvea, including spindle cell melanoma, mixed cell melanoma and epithelioid cell melanoma; (e) cancers of the orbit, including sarcoma of the orbit, soft tissue tumor, and sarcoma of the bone; and (f) retinoblastoma.

In one embodiment of the present invention, the agent may be formulated into a formulation selected from the group consisting of a tablet, a capsule, an injection, a troche, a powder, a granule, a solution, a suspension, an oral solution, an emulsion, a syrup, a suppository, a vaginal tablet and a pill, but is not limited thereto.

In one embodiment of the present invention, the present invention provides a combination kit for preventing or treating cancer comprising complexed, mixed or combined preparation of an anti-viral agent; and an antidepressant.

In one embodiment of the present invention, the present invention provides a combination kit for preventing or treating cancer, comprising the complex, mixed or combined preparation, and the kit comprising an anti-viral agent and an antidepressant, and a biguanide-based compound or a pharmaceutically acceptable salt thereof.

In one embodiment of the present invention, the present invention provides a method of treating cancer comprising the step of administering in complex, mixture, or combination a preparation containing a pharmaceutically effective amount of an antiviral agent and a preparation containing a pharmaceutically effective amount of an antidepressant to a subject with cancer.

In one embodiment of the present invention, the present invention provides a method of treating cancer comprising the step of administering in complex, mixture, or combination a preparation containing a pharmaceutically effective amount of an antiviral agent, a preparation containing a pharmaceutically effective amount of an antidepressant, and a preparation containing a biguanide-based compound or a pharmaceutically acceptable salt thereof to a subject with cancer.

In one embodiment of the present invention provides a complex, mixed or combined preparation for use in the prevention or treatment of cancer, comprising a preparation containing an antiviral agent and a preparation containing an antidepressant.

In one embodiment of the present invention provides a complex, mixed or combined preparation for use in the prevention or treatment of cancer, comprising a preparation containing an antiviral agent, a preparation containing an antidepressant, and a preparation containing a biguanide-based compound or a pharmaceutically acceptable salt thereof.

According to the present invention, while the antiviral agent, antidepressant, and biguanide-based compound or its pharmaceutically acceptable salt show minimal anti-cancer effects when administered alone, significant anti-cancer effects have been observed in various types of cancer when administered in combination, mixture, or co-administration. Therefore, pharmaceutical compositions containing these as active ingredients can be useful for cancer prevention or treatment.

### [Brief description of the drawings]

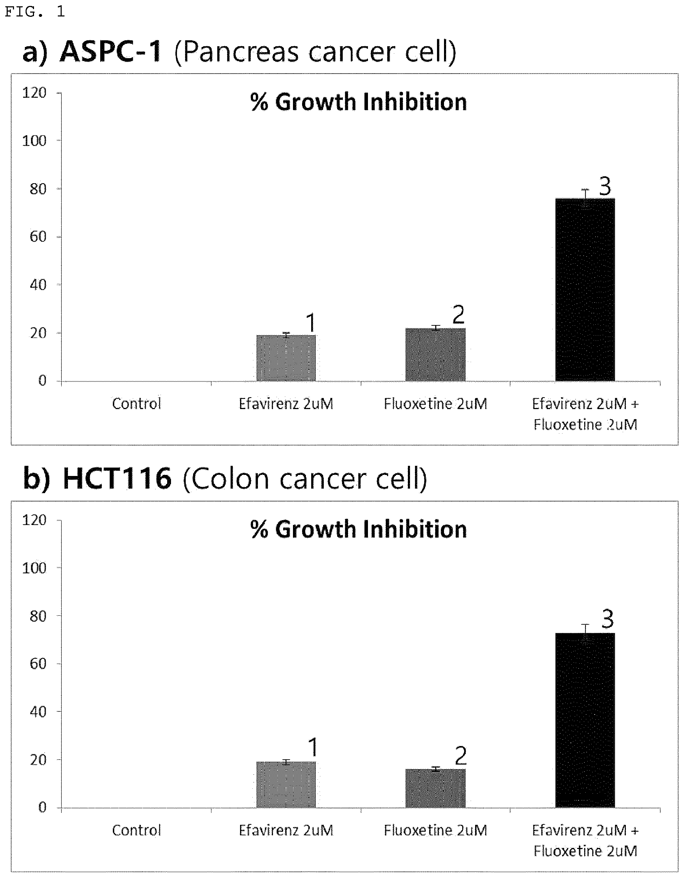

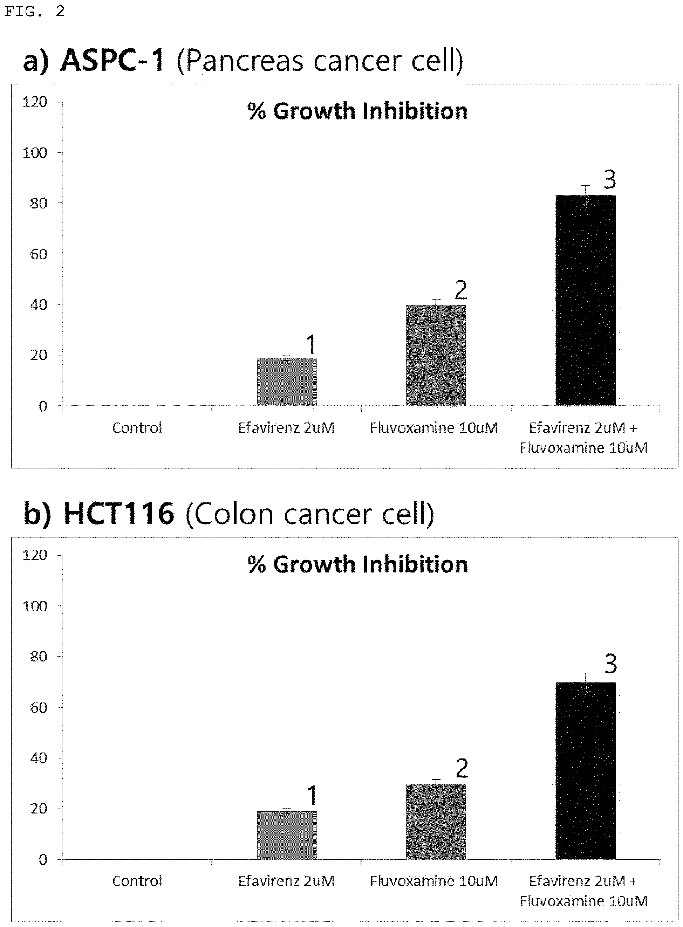

Figure 1 shows the growth inhibition (%) of a) pancreatic cancer cell lines and b) colorectal cancer cell lines when treated for 24 hours with 2 µM efavirenz, 2 µM fluoxetine, and 2 µM efavirenz + 2 µM fluoxetine;
   a) Pancreatic cancer cell line ASPC-1
      Blue bar graph (1): Percent growth inhibition of cancer cells treated with 2 µM efavirenz alone;
      Green bar graph (2): Percent growth inhibition of cancer cells treated with 2 µM fluoxetine alone; and
      Red bar graph (3): Percent growth inhibition of cancer cells treated with a combination of 2 µM efavirenz and 2 µM fluoxetine.
   b) Colorectal cancer cell line HCT116
      Blue bar graph (1): Percent growth inhibition of cancer cells when treated with 2 µM of efavirenz alone;
      Green bar graph (2): Percent growth inhibition of cancer cells when treated with 2 µM of fluoxetine alone; and
      Red bar graph (3): Percent growth inhibition of cancer cells when treated with a combination of 2 µM efavirenz and 2 µM fluoxetine
Figure 2 shows the growth inhibition (%) of a) pancreatic cancer cells and b) colorectal cancer cells when treated with 2 µM efavirenz, 10 µM fluvoxamine, and a combination of 2 µM efavirenz + 10 µM fluvoxamine for 24 hours.
   a) Pancreatic cancer cell line ASPC-1
      Blue bar graph (1): % growth inhibition of cancer cells treated with 2 µM efavirenz alone;
      Green bar graph (2): % growth inhibition of cancer cells treated with 10 µM fluvoxamine alone; and
      Red bar graph (3): % growth inhibition of cancer cells treated with a combination of 2 µM efavirenz and 10 µM fluvoxamine.
   b) Colorectal cancer cell line HCT116
      Blue bar graph (1): % growth inhibition of cancer cells treated with 2 µM efavirenz alone;
      Green bar graph (2): % growth inhibition of cancer cells treated with 10 µM fluvoxamine alone; and
      Red bar graph (3): % growth inhibition of cancer cells treated with a combination of 2 µM efavirenz and 10 µM fluvoxamine.

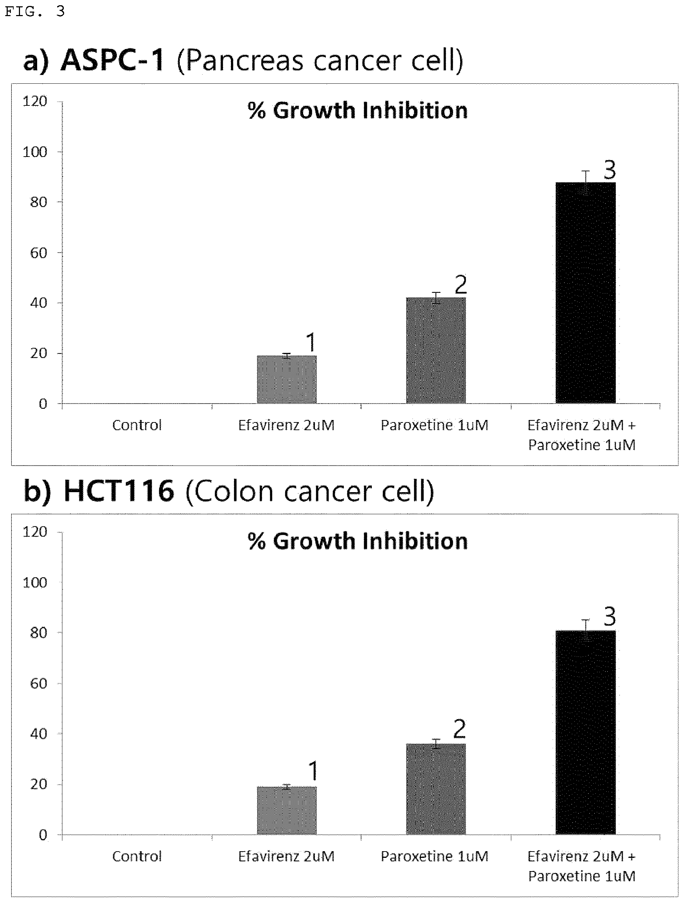

Figure 3 shows the growth inhibition (%) of a) pancreatic cancer cell line and b) colorectal cancer cell line when treated for 24 hours with 2 µM efavirenz, 1 µM paroxetine, and 2 µM efavirenz + 1 µM paroxetine;
   a) Pancreatic cancer cell line ASPC-1
      Blue bar (1): % growth inhibition of cancer cells treated with 2 µM efavirenz alone;
      Green bar (2): % growth inhibition of cancer cells treated with 1 µM paroxetine alone; and
      Red bar (3): % growth inhibition of cancer cells treated with 2 µM efavirenz and 1 µM paroxetine in combination.
   b) colorectal cancer cell line HCT116
      Blue bar (1): % growth inhibition of cancer cells treated with 2 µM efavirenz alone;
      Green bar (2): % growth inhibition of cancer cells treated with 1 µM paroxetine alone; and
      Red bar (3): % growth inhibition of cancer cells treated with 2 µM efavirenz and 1 µM paroxetine in combination.

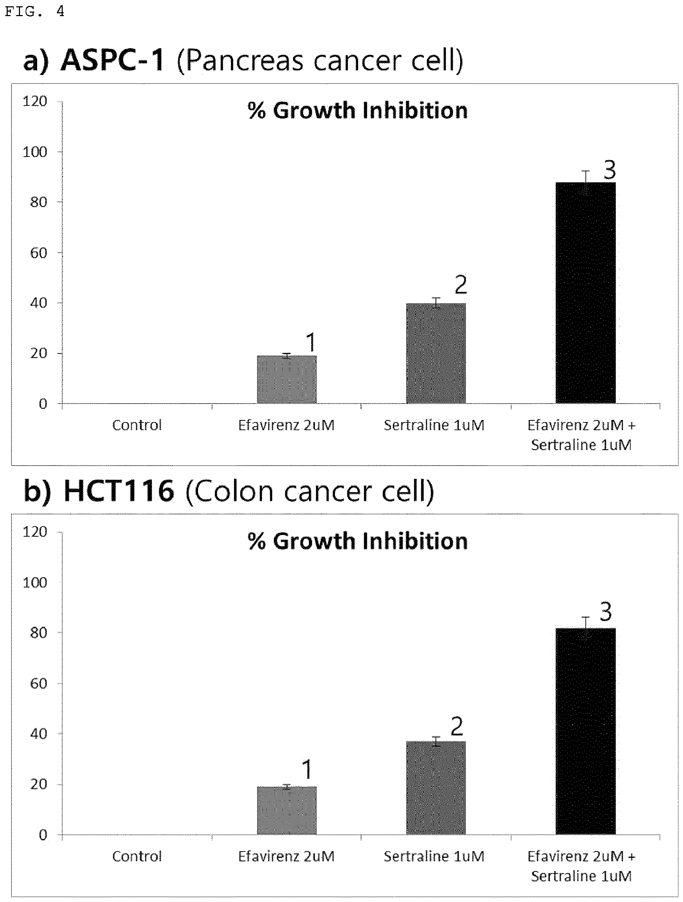

Figure 4 shows the growth inhibition (%) of a) pancreatic cancer cell line and b) colorectal cancer cell line when treated for 24 hours with 2 µM efavirenz, 1 µM sertraline, and 2 µM efavirenz + 1 µM sertraline;
   a) Pancreatic cancer cell line ASPC-1
      Blue bar (1): % growth inhibition of cancer cells treated with 2 µM efavirenz alone;
      Green bar (2): % growth inhibition of cancer cells treated with 1 µM sertraline alone; and
      Red bar (3): % growth inhibition of cancer cells treated with 2 µM efavirenz and 1 µM sertraline in combination.
   b) colorectal cancer cell line HCT116
      Blue bar (1): % growth inhibition of cancer cells treated with 2 µM efavirenz alone;
      Green bar (2): % growth inhibition of cancer cells treated with 1 µM sertraline alone; and
      Red bar (3): % growth inhibition of cancer cells treated with 2 µM efavirenz and 1 µM sertraline in combination.

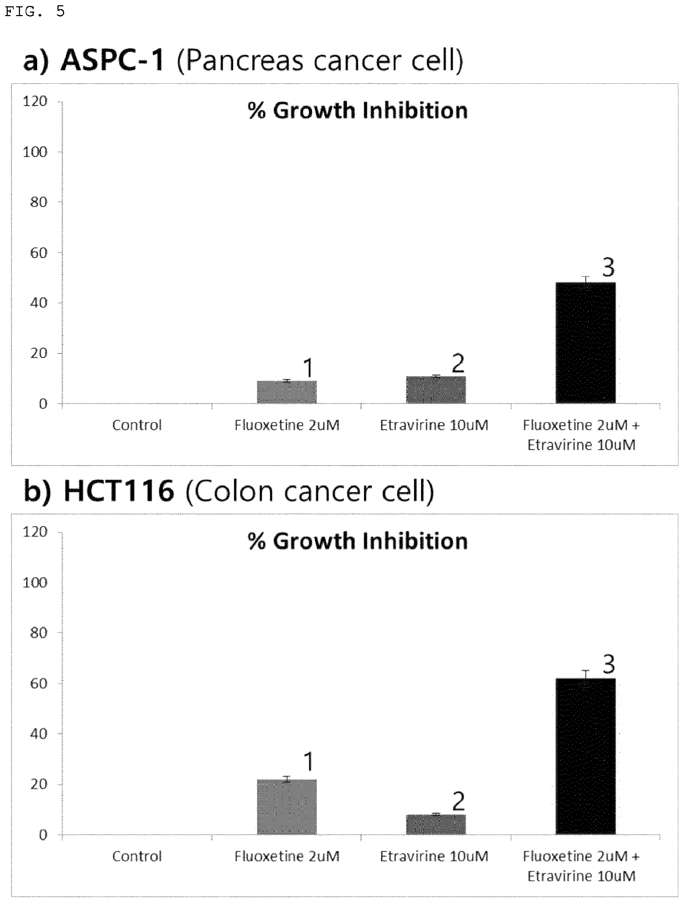

Figure 5 shows the growth inhibition (%) of a) pancreatic cancer cell line and b) colorectal cancer cell line when treated for 24 hours with 10 µM etravirine, 2 µM fluoxetine, and 10 µM etravirine + 2 µM fluoxetine;
   a) Pancreatic cancer cell line ASPC-1
      Blue bar (1): % growth inhibition of cancer cells treated with 10 µM etravirine alone;
      Green bar (2): % growth inhibition of cancer cells treated with 2 µM fluoxetine alone; and
      Red bar (3): % growth inhibition of cancer cells treated with 10 µM etravirine and 2 µM fluoxetine in combination.
   b) colorectal cancer cell line HCT116
      Blue bar (1): % growth inhibition of cancer cells treated with 10 µM etravirine alone;
      Green bar (2): % growth inhibition of cancer cells treated with 2 µM fluoxetine alone; and
      Red bar (3): % growth inhibition of cancer cells treated with 10 µM etravirine and 2 µM fluoxetine in combination.
Figure 6 shows the growth inhibition (%) of colorectal cancer cell line HCT116 when treated for 24 hours with 2 µM rilpivirine, 2 µM fluoxetine, and 2 µM rilpivirine + 2 µM fluoxetine;
   Blue bar (1): % growth inhibition of cancer cells treated with 2 µM rilpivirine alone;
   Green bar (2): % growth inhibition of cancer cells treated with 2 µM fluoxetine alone; and
   Red bar (3): % growth inhibition of cancer cells treated with 2 µM rilpivirine and 2 µM fluoxetine in combination.
Figure 7 shows the growth inhibition (%) of a) pancreatic cancer cell line and b) colorectal cancer cell line when treated for 24 hours with 2 µM efavirenz, 10 µM duloxetine, and 2 µM efavirenz + 10 µM duloxetine;
   a) Pancreatic cancer cell line ASPC-1
      Blue bar (1): % growth inhibition of cancer cells treated with 2 µM efavirenz alone;
      Green bar (2): % growth inhibition of cancer cells treated with 10 µM duloxetine alone; and
      Red bar (3): % growth inhibition of cancer cells treated with 2 µM efavirenz and 10 µM duloxetine in combination.
   b) Colorectal cancer cell line HCT116
      Blue bar (1): % growth inhibition of cancer cells treated with 2 µM efavirenz alone;
      Green bar (2): % growth inhibition of cancer cells treated with 10 µM duloxetine alone; and
      Red bar (3): % growth inhibition of cancer cells treated with 2 µM efavirenz and 10 µM duloxetine in combination.

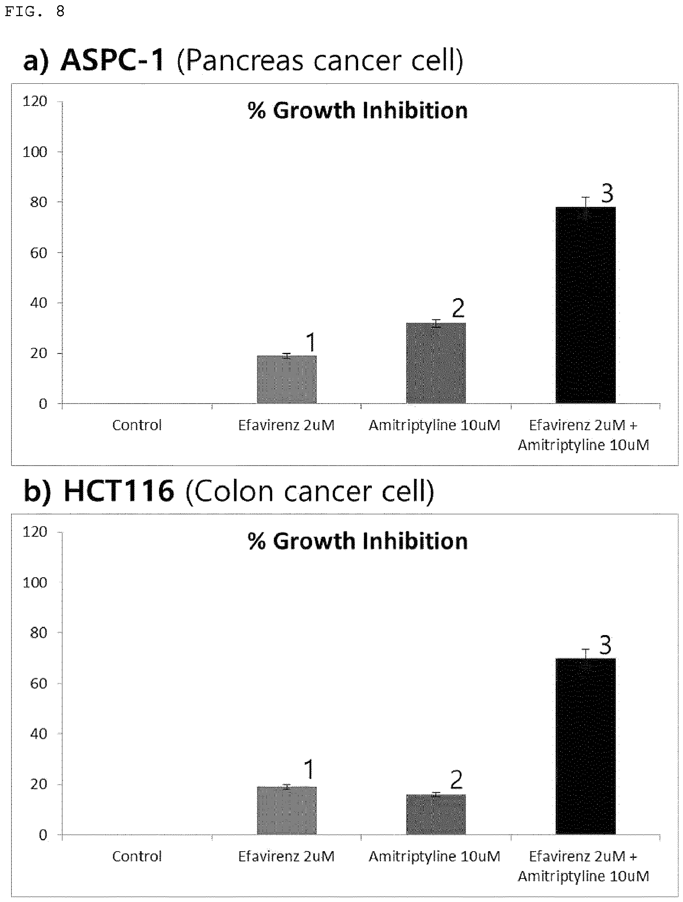

Figure 8 shows the growth inhibition (%) of a) pancreatic cancer cell line and b) colorectal cancer cell line when treated for 24 hours with 2 µM efavirenz, 10 µM amitriptyline, and 2 µM efavirenz + 10 µM amitriptyline;
   a) Pancreatic cancer cell line ASPC-1
      Blue bar (1): % growth inhibition of cancer cells treated with 2 µM efavirenz alone;
      Green bar (2): % growth inhibition of cancer cells treated with 10 µM amitriptyline alone; and
      Red bar (3): % growth inhibition of cancer cells treated with 2 µM efavirenz and 10 µM amitriptyline in combination.
   b) Colorectal cancer cell line HCT116
      Blue bar (1): % growth inhibition of cancer cells treated with 2 µM efavirenz alone;
      Green bar (2): % growth inhibition of cancer cells treated with 10 µM amitriptyline alone; and
      Red bar (3): % growth inhibition of cancer cells treated with 2 µM efavirenz and 10 µM amitriptyline in combination.

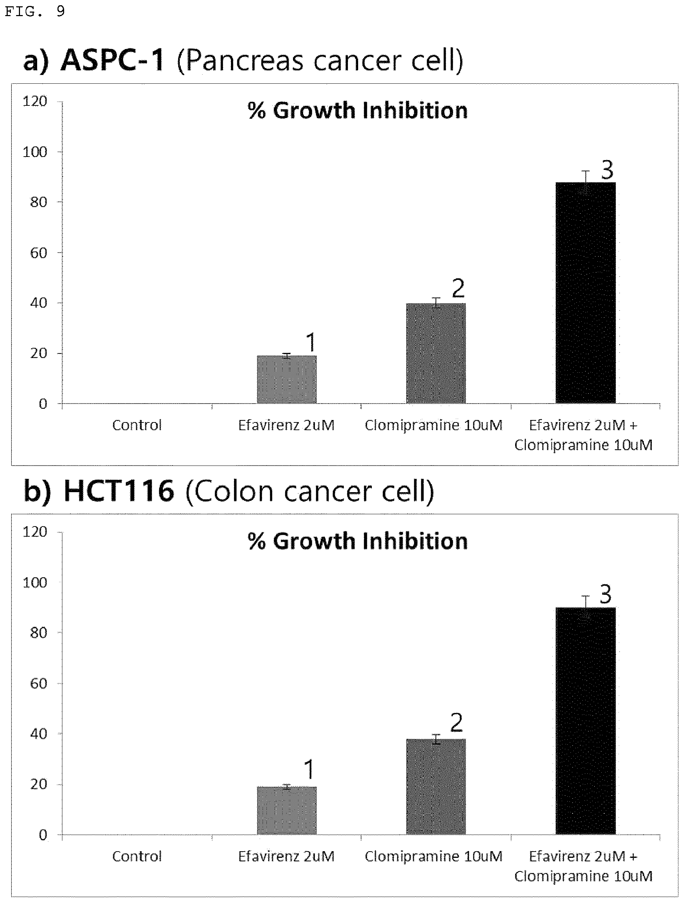

Figure 9 shows the growth inhibition (%) of a) pancreatic cancer cell line and b) colorectal cancer cell line when treated for 24 hours with 2 µM efavirenz, 10 µM clomipramine, and 2 µM efavirenz + 10 µM clomipramine;
   a) Pancreatic cancer cell line ASPC-1
      Blue bar (1): % growth inhibition of cancer cells treated with 2 µM efavirenz alone;
      Green bar (2): % growth inhibition of cancer cells treated with 10 µM clomipramine alone; and
      Red bar (3): % growth inhibition of cancer cells treated with 2 µM efavirenz and 10 µM clomipramine in combination.
   b) Colorectal cancer cell line HCT116
      Blue bar (1): % growth inhibition of cancer cells treated with 2 µM efavirenz alone;
      Green bar (2): % growth inhibition of cancer cells treated with 10 µM clomipramine alone; and
      Red bar (3): % growth inhibition of cancer cells treated with 2 µM efavirenz and 10 µM clomipramine in combination.

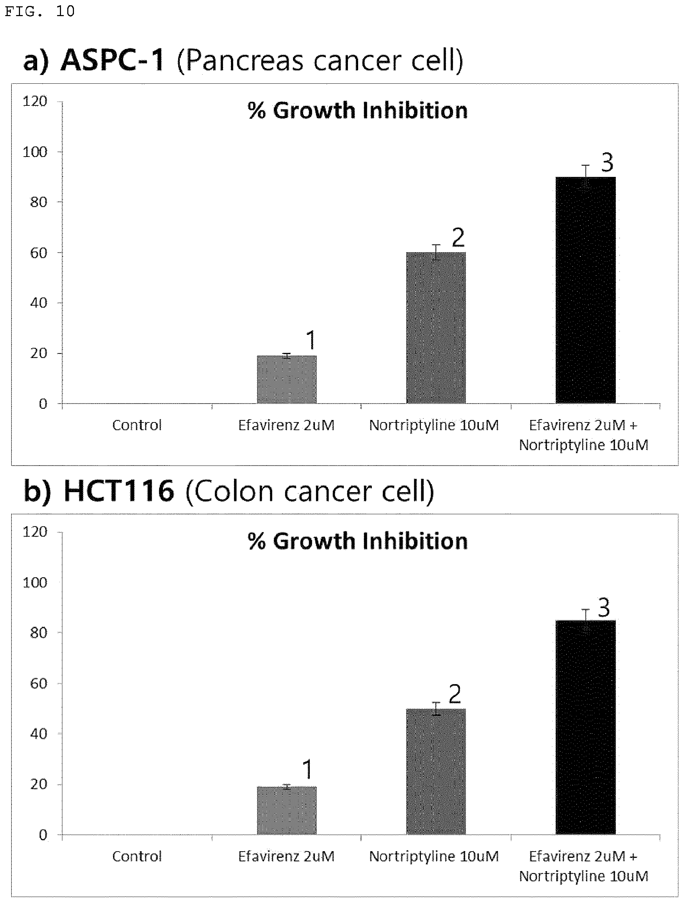

Figure 10 shows the growth inhibition (%) of a) pancreatic cancer cell line and b) colorectal cancer cell line when treated for 24 hours with 2 µM efavirenz, 10 µM nortriptyline, and 2 µM efavirenz + 10 µM nortriptyline;
   a) Pancreatic cancer cell line ASPC-1
      Blue bar (1): % growth inhibition of cancer cells treated with 2 µM efavirenz alone;
      Green bar (2): % growth inhibition of cancer cells treated with 10 µM nortriptyline alone; and
      Red bar (3): % growth inhibition of cancer cells treated with 2 µM efavirenz and 10 µM nortriptyline in combination.
   b) Colorectal cancer cell line HCT116
      Blue bar (1): % growth inhibition of cancer cells treated with 2 µM efavirenz alone;
      Green bar (2): % growth inhibition of cancer cells treated with 10 µM nortriptyline alone; and
      Red bar (3): % growth inhibition of cancer cells treated with 2 µM efavirenz and 10 µM nortriptyline in combination.

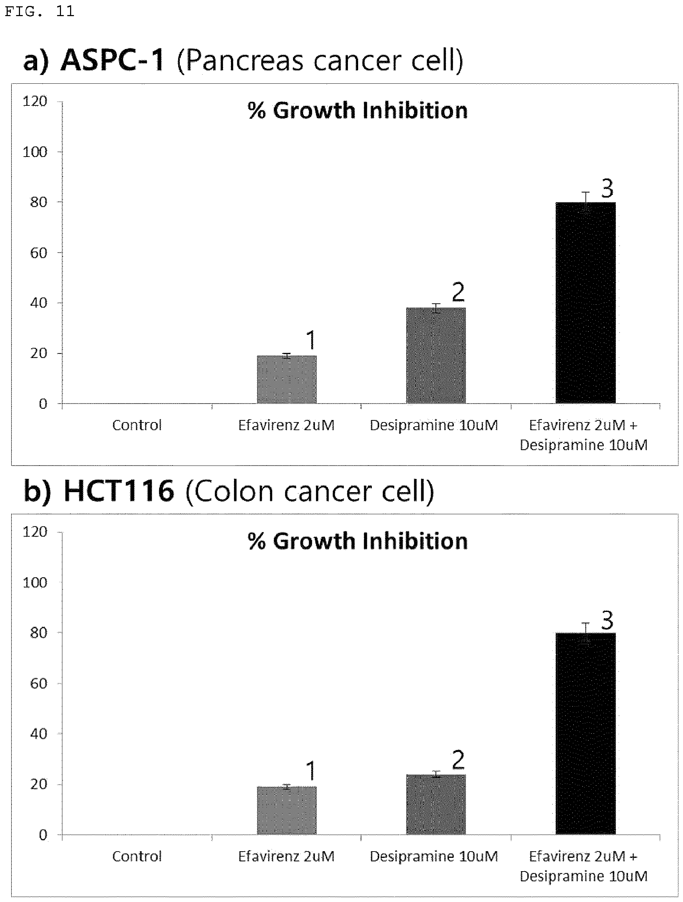

Figure 11 shows the growth inhibition (%) of a) pancreatic cancer cell line and b) colorectal cancer cell line when treated for 24 hours with 2 µM efavirenz, 10 µM desipramine, and 2 µM efavirenz + 10 µM desipramine;
   a) Pancreatic cancer cell line ASPC-1
      Blue bar (1): % growth inhibition of cancer cells treated with 2 µM efavirenz alone;
      Green bar (2): % growth inhibition of cancer cells treated with 10 µM desipramine alone; and
      Red bar (3): % growth inhibition of cancer cells treated with 2 µM efavirenz and 10 µM desipramine in combination.
   b) Colorectal cancer cell line HCT116
      Blue bar (1): % growth inhibition of cancer cells treated with 2 µM efavirenz alone;
      Green bar (2): % growth inhibition of cancer cells treated with 10 µM desipramine alone; and
      Red bar (3): % growth inhibition of cancer cells treated with 2 µM efavirenz and 10 µM desipramine in combination.

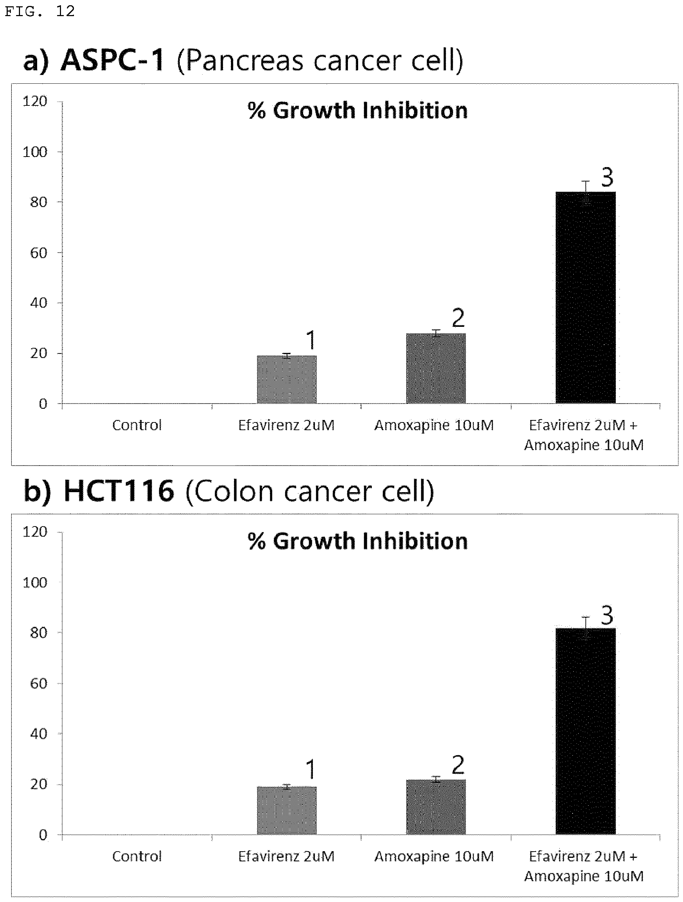

Figure 12 shows the growth inhibition (%) of a) pancreatic cancer cell line and b) colorectal cancer cell line when treated for 24 hours with 2 µM efavirenz, 10 µM amoxapine, and 2 µM efavirenz + 10 µM amoxapine;
   a) Pancreatic cancer cell line ASPC-1
      Blue bar (1): % growth inhibition of cancer cells treated with 2 µM efavirenz alone;
      Green bar (2): % growth inhibition of cancer cells treated with 10 µM amoxapine alone; and
      Red bar (3): % growth inhibition of cancer cells treated with 2 µM efavirenz and 10 µM amoxapine in combination.
   b) Colorectal cancer cell line HCT116
      Blue bar (1): % growth inhibition of cancer cells treated with 2 µM efavirenz alone;
      Green bar (2): % growth inhibition of cancer cells treated with 10 µM amoxapine alone; and
      Red bar (3): % growth inhibition of cancer cells treated with 2 µM efavirenz and 10 µM amoxapine in combination.

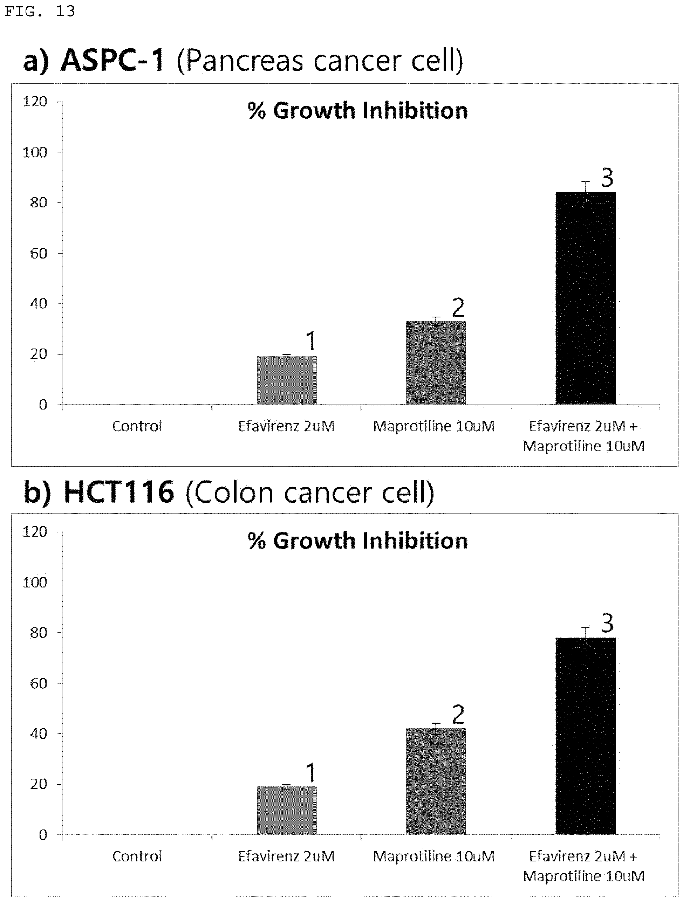

Figure 13 shows the growth inhibition (%) of a) pancreatic cancer cell line and b) colorectal cancer cell line when treated for 24 hours with 2 µM efavirenz, 10 µM maprotiline, and 2 µM efavirenz + 10 µM maprotiline;
   a) Pancreatic cancer cell line ASPC-1
      Blue bar (1): % growth inhibition of cancer cells treated with 2 µM efavirenz alone;
      Green bar (2): % growth inhibition of cancer cells treated with 10 µM maprotiline alone; and
      Red bar (3): % growth inhibition of cancer cells treated with 2 µM efavirenz and 10 µM maprotiline in combination.
   b) Colorectal cancer cell line HCT116
      Blue bar (1): % growth inhibition of cancer cells treated with 2 µM efavirenz alone;
      Green bar (2): % growth inhibition of cancer cells treated with 10 µM maprotiline alone; and
      Red bar (3): % growth inhibition of cancer cells treated with 2 µM efavirenz and 10 µM maprotiline in combination.

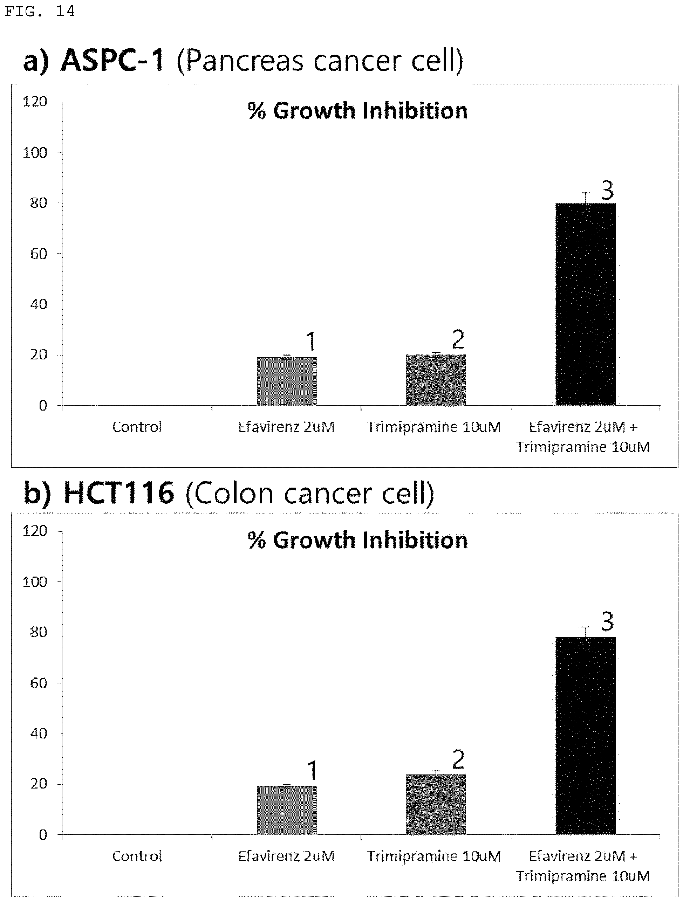

Figure 14 shows the growth inhibition (%) of a) pancreatic cancer cell line and b) colorectal cancer cell line when treated for 24 hours with 2 µM efavirenz, 10 µM trimipramine, and 2 µM efavirenz + 10 µM trimipramine;
   a) Pancreatic cancer cell line ASPC-1
      Blue bar (1): % growth inhibition of cancer cells treated with 2 µM efavirenz alone;
      Green bar (2): % growth inhibition of cancer cells treated with 10 µM trimipramine alone; and
      Red bar (3): % growth inhibition of cancer cells treated with 2 µM efavirenz and 10 µM trimipramine in combination.
   b) Colorectal cancer cell line HCT116
      Blue bar (1): % growth inhibition of cancer cells treated with 2 µM efavirenz alone;
      Green bar (2): % growth inhibition of cancer cells treated with 10 µM trimipramine alone; and
      Red bar (3): % growth inhibition of cancer cells treated with 2 µM efavirenz and 10 µM trimipramine in combination.

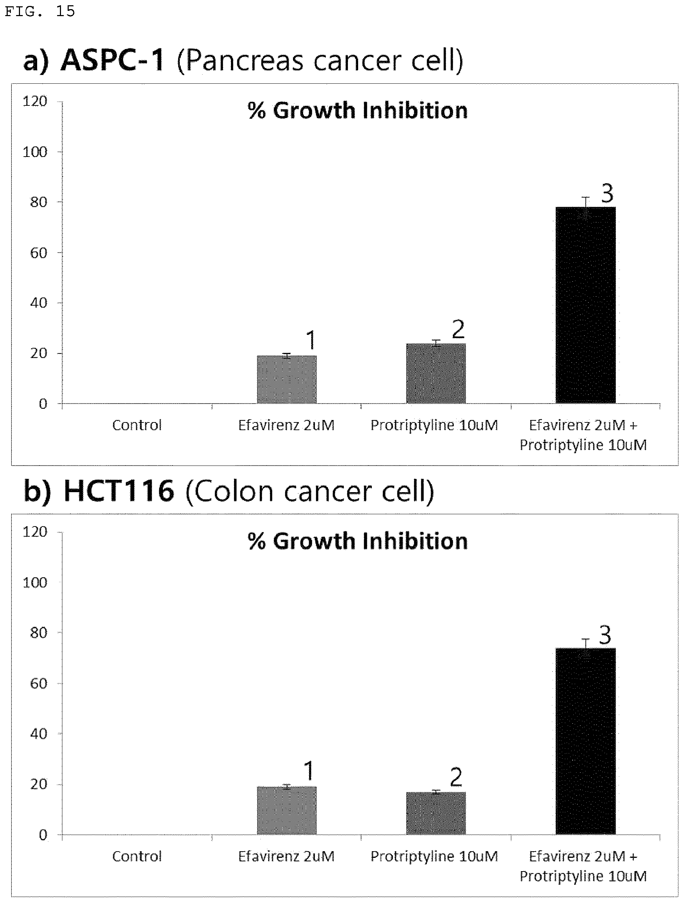

Figure 15 shows the growth inhibition (%) of a) pancreatic cancer cell line and b) colorectal cancer cell line when treated for 24 hours with 2 µM efavirenz, 10 µM protriptyline, and 2 µM efavirenz + 10 µM protriptyline;
   a) Pancreatic cancer cell line ASPC-1
      Blue bar (1): % growth inhibition of cancer cells treated with 2 µM efavirenz alone;
      Green bar (2): % growth inhibition of cancer cells treated with 10 µM protriptyline alone; and
      Red bar (3): % growth inhibition of cancer cells treated with 2 µM efavirenz and 10 µM protriptyline in combination.
   b) Colorectal cancer cell line HCT116
      Blue bar (1): % growth inhibition of cancer cells treated with 2 µM efavirenz alone;
      Green bar (2): % growth inhibition of cancer cells treated with 10 µM protriptyline alone; and
      Red bar (3): % growth inhibition of cancer cells treated with 2 µM efavirenz and 10 µM protriptyline in combination.

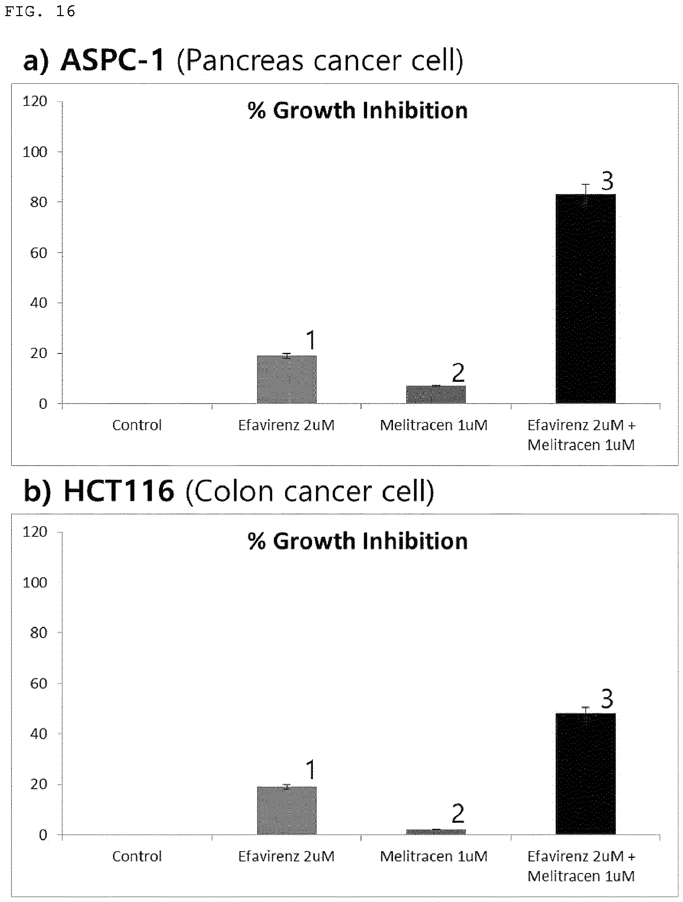

Figure 16 shows the growth inhibition (%) of a) pancreatic cancer cell line and b) colorectal cancer cell line when treated for 24 hours with 2 µM efavirenz, 1 µM melitracen, and 2 µM efavirenz + 1 µM melitracen;
   a) Pancreatic cancer cell line ASPC-1
      Blue bar (1): % growth inhibition of cancer cells treated with 2 µM efavirenz alone;
      Green bar (2): % growth inhibition of cancer cells treated with 1 µM melitracen alone; and
      Red bar (3) : % growth inhibition of cancer cells treated with 2 µM efavirenz and 1 µM melitracen in combination.
   b) Colorectal cancer cell line HCT116
      Blue bar (1): % growth inhibition of cancer cells treated with 2 µM efavirenz alone;
      Green bar (2): % growth inhibition of cancer cells treated with 1 µM melitracen alone; and
      Red bar (3): % growth inhibition of cancer cells treated with 2 µM efavirenz and 1 µM melitracen in combination.

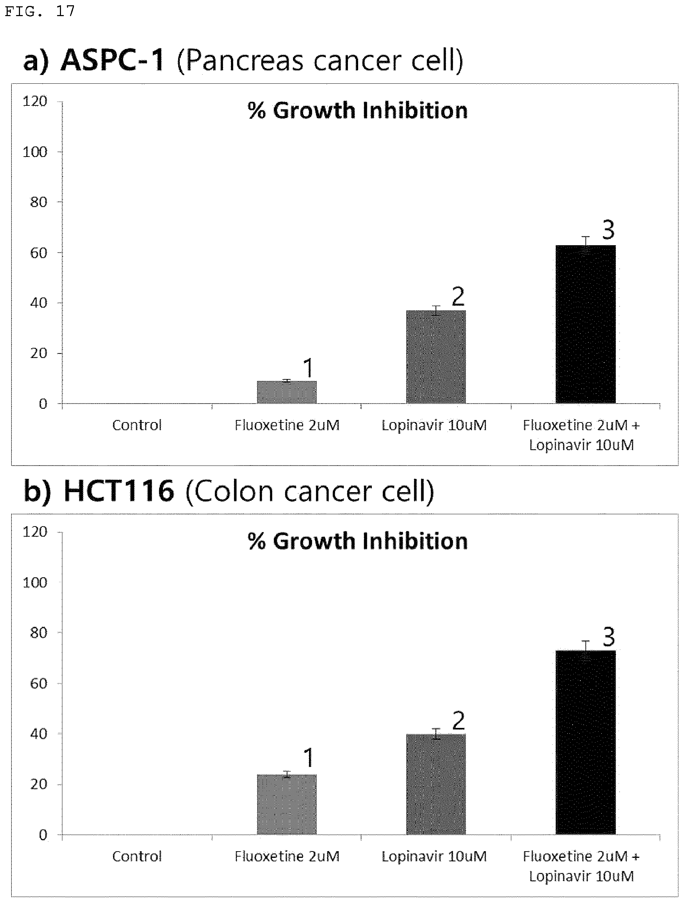

Figure 17 shows the growth inhibition (%) of a) pancreatic cancer cell line and b) colorectal cancer cell line when treated for 24 hours with 10 µM lopinavir, 2 µM fluoxetine, and 10 µM lopinavir + 2 µM fluoxetine;
   a) Pancreatic cancer cell line ASPC-1
      Blue bar (1): % growth inhibition of cancer cells treated with 10 µM lopinavir alone;
      Green bar (2): % growth inhibition of cancer cells treated with 2 µM fluoxetine alone; and
      Red bar (3): % growth inhibition of cancer cells treated with 10 µM lopinavir and 2 µM fluoxetine in combination.
   b) Colorectal cancer cell line HCT116
      Blue bar (1): % growth inhibition of cancer cells treated with 10 µM lopinavir alone;
      Green bar (2): % growth inhibition of cancer cells treated with 2 µM fluoxetine alone; and
      Red bar (3): % growth inhibition of cancer cells treated with 10 µM lopinavir and 2 µM fluoxetine in combination.

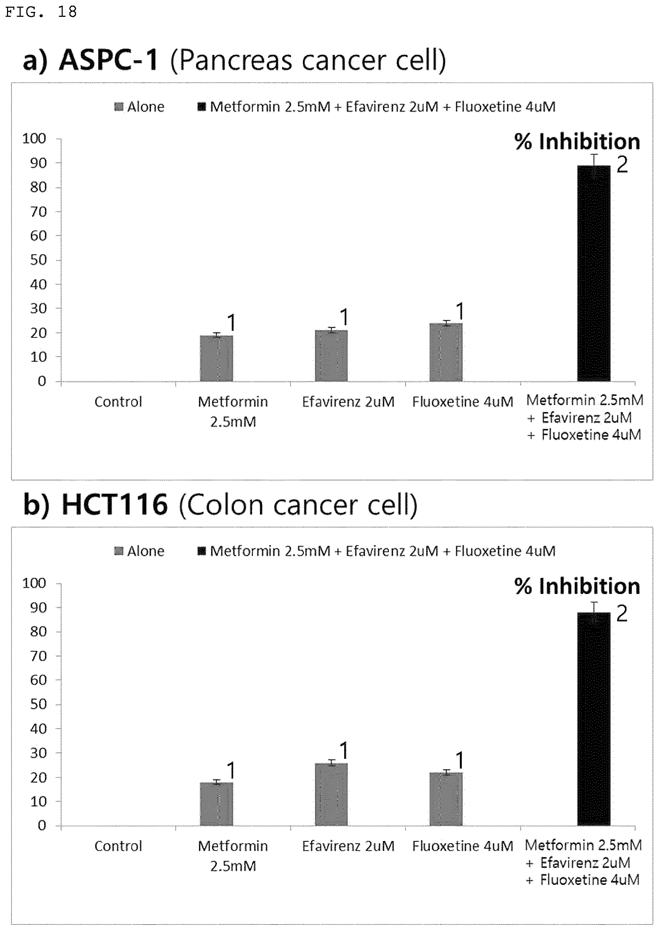

Figure 18 shows the growth inhibition (%) of a) pancreatic cancer cell line and b) colorectal cancer cell line when treated for 24 hours with 2 µM efavirenz, 4 µM fluoxetine, and 2.5 mM metformin;
   a) Pancreatic cancer cell line ASPC-1
      Blue bar (1): % growth inhibition of cancer cells treated with 2 µM efavirenz, 4 µM fluoxetine, and 2.5 mM metformin alone;
      Red bar (3): % growth inhibition of cancer cells treated with 2 µM efavirenz, 4 µM fluoxetine, and 2.5 mM metformin in combination.
   b) Colorectal cancer cell line HCT116
      Blue bar (1): % growth inhibition of cancer cells treated with 2 µM efavirenz, 4 µM fluoxetine, and 2.5 mM metformin alone;
      Red bar (3): % growth inhibition of cancer cells treated with 2 µM efavirenz, 4 µM fluoxetine, and 2.5 mM metformin in combination.

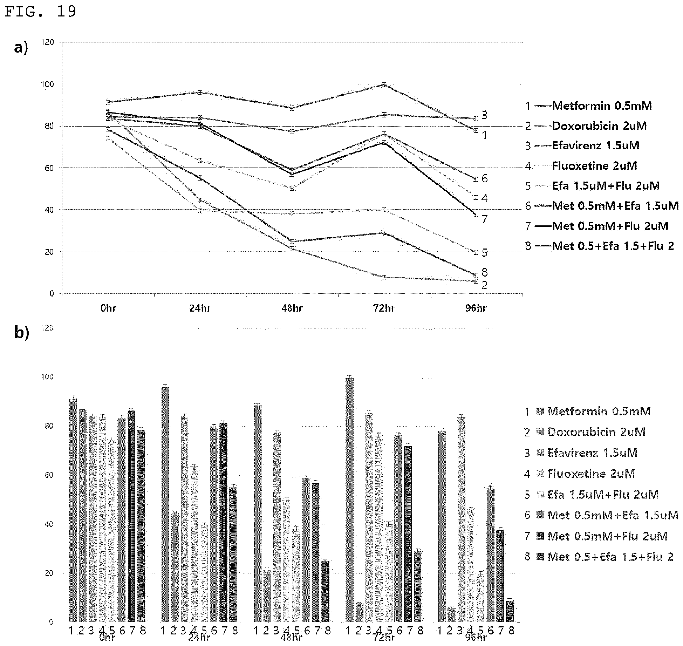

Figure 19 shows the growth inhibition (%) of colorectal cancer cell line HCT116 when treated with 0.5 mM metformin, 2 µM doxorubicin, 1.5 µM efavirenz, 2 µM fluoxetine, 1.5 µM efavirenz and 2 µM fluoxetine; 1.5 µM efavirenz and 0.5 mM metformin; 2 µM fluoxetine and 0.5 mM metformin; and 1.5 µM efavirenz, 2 µM fluoxetine and 0.5 mM metformin for 0, 24, 48, 72 and 96 hours.
   a) Blue bar (1): % growth inhibition of cancer cells treated with 0.5 mM metformin alone for 0, 24, 48, 72 and 96 hours;
      Orange bar (2): % growth inhibition of cancer cells treated with 2 µM doxorubicin alone for 0, 24, 48, 72 and 96 hours;
      Grey bar (3): % growth inhibition of cancer cells treated with 1.5 µM efavirenz alone for 0, 24, 48, 72 and 96 hours;
      Yellow bar (4): % growth inhibition of cancer cells treated with 2 µM fluoxetine alone for 0, 24, 48, 72 and 96 hours;
      Skyblue bar (5): % growth inhibition of cancer cells treated with 1.5 µM efavirenz and 2 µM fluoxetine in combination for 0, 24, 48, 72 and 96 hours;
      Green bar (6): % growth inhibition of cancer cells treated with 1.5 µM efavirenz and 0.5 mM metformin in combination for 0, 24, 48, 72 and 96 hours;
      Navy bar (7): % growth inhibition of cancer cells treated with 2 µM fluoxetine and 0.5 mM metformin in combination for 0, 24, 48, 72 and 96 hours;
      Brown bar (8): % growth inhibition of cancer cells treated with 1.5 µM efavirenz, 2 µM fluoxetine and 0.5 mM metformin in combination for 0, 24, 48, 72 and 96 hours;
   b) Blue bar (1): % growth inhibition of cancer cells treated with 0.5 mM metformin alone for 0, 24, 48, 72 and 96 hours;
      Orange bar (2): % growth inhibition of cancer cells treated with 2 µM doxorubicin alone for 0, 24, 48, 72 and 96 hours;
      Grey bar (3): % growth inhibition of cancer cells treated with 1.5 µM efavirenz alone for 0, 24, 48, 72 and 96 hours;
      Yellow bar (4): % growth inhibition of cancer cells treated with 2 µM fluoxetine alone for 0, 24, 48, 72 and 96 hours;
      Skyblue bar (5): % growth inhibition of cancer cells treated with 1.5 µM efavirenz and 2 µM fluoxetine in combination for 0, 24, 48, 72 and 96 hours;
      Green bar (6): % growth inhibition of cancer cells treated with 1.5 µM efavirenz and 0.5 mM metformin in combination for 0, 24, 48, 72 and 96 hours;
      Navy bar (7): % growth inhibition of cancer cells treated with 2 µM fluoxetine and 0.5 mM metformin in combination for 0, 24, 48, 72 and 96 hours;
      Brown bar (8): % growth inhibition of cancer cells treated with 1.5 µM efavirenz, 2 µM fluoxetine and 0.5 mM metformin in combination for 0, 24, 48, 72 and 96 hours;

### [Description of Embodiments]

Hereafter, the present invention will be described in more detail.

The present invention relates to a complex, mixed, or combined pharmaceutical preparation in use, for preventing or treating cancer, comprising a first component including an anti-viral agent; and a second component including an antidepressant as active ingredients of the complex, mixed or combined preparation:
a method for preventing or treating cancer comprising administering pharmaceutically effective amounts of a first component including an anti-viral agent; and a second component including an antidepressant to an individual in a complexed, mixed, or combined manner, and
a preparation for preventing or treating cancer comprising a first component including an anti-viral agent; and a second component including an antidepressant in a complexed, mixed, or combined manner.

The present invention also relates to a pharmaceutical composition, for preventing or treating cancer, comprising a first component including an anti-viral agent; a second component including an antidepressant; and a third component including a biguanide-based compound or a pharmaceutically acceptable salt thereof further added, as active ingredients for use in the form of a complexed, mixed or combined preparation:
a method for preventing or treating cancer comprising administering pharmaceutically effective amounts of a first component including an anti-viral agent; a second component including an antidepressant; and a third component including a biguanide-based compound or a pharmaceutically acceptable salt thereof to an individual in a complexed, mixed or combined manner; and
a preparation for preventing or treating cancer comprising a first component including an anti-viral agent; a second component including an antidepressant; and a third component including a biguanide-based compound or a pharmaceutically acceptable salt thereof in a complexed, mixed, or combined manner.

In the present invention, "prevention" means any action that suppresses the onset of disease or delays the onset of disease by administration of the composition. In the present invention, "improvement" or "treatment" means any action that improves or advantageously changes the symptoms of the disease by administration of the composition.

In the present invention, "administration" means providing a given substance to a patient by any suitable method, and the composition of the present invention may be administered orally or parenterally through all general administration routes as long as it can reach the target tissue. The composition may be administered in a form mounted on any device capable of transporting the active substance to a target cell.

In the present invention, the anti-viral agent may be NNRTIs(Non-nucleoside reverse-transcriptase inhibitors) family compound, or its derivative, or a pharmaceutically acceptable salt thereof, and the NNRTI family compound or its derivative may be selected from the group consisting of efavirenz, etravirine, nevirapine, doravirine, rilpivirine, and delavirdine.

In the present invention, the anti-viral agent may be also NRTIs(Nucleoside reverse-transcriptase inhibitors) family compound or its derivative, or a pharmaceutically acceptable salt thereof, and the NRTI family compound or its derivative may be selected from the group consisting of zidovudine, didanosine, zalcitabine, stavudine, lamivudine, abacavir, tenofovir disoproxil fumarate, emtricitabine, tenofovir alafenamide fumarate.

In the present invention, the anti-viral agent may be also selected from PIs(Protease inhibitors) family compound, or its derivative, or a pharmaceutically acceptable salt thereof, and the PIs family compound or its derivative may be selected from the group consisting of saquinavir, ritonavir, indinavir, nelfinavir, amprenavir, lopinavir, atazanavir, fosamprenavir, tipranavir, darunavir, as for monotherapy, and atazanavir+cobicistat, dadrunavir+ cobicistat, ritonavir+lopinavir, as for combination therapy.

In the present invention, the anti-viral agent may be also INSTI(Integrase Strand Transfer Inhibitor) family compound, or its derivative, or a pharmaceutically acceptable salt thereof, and the INSTI family compound or its derivative may be dolutegravir and raltegravir.

In the present invention, the anti-viral agent may be also fusion inhibitor family compound or its derivative, or a pharmaceutically acceptable salt thereof, and the fusion inhibitor compound may be enfuvirtide.

In the present invention, the anti-viral agent may be also CCR5 (Chemokine (C-C motif) ligand 5) inhibitor family compound or its derivative, or a pharmaceutically acceptable salt thereof, and the CCR5 inhibitor compound or its derivative may be selected from the group consisting of maraviroc as for monotherapy, cobicistat/elvitegravir/emtricitabine/Tenofovir alafenamide, cobicistat/Elvitegravir/emtricitabine/Tenofovir disoproxil, and abacavir/dolutegravir/lamivudine as for combination therapy.

In addition, the anti-viral agent according to the present invention may be a therapeutic agent for influenza in the treatment of influenza A and influenza B virus infections, wherein the anti-viral agent may be an uncoating inhibitor family compound, or its derivative, or a pharmaceutically acceptable salt thereof, such as amantadine, rimantadine, and NAIs(Neuraminidase inhibitors) family compound or its derivative, or a pharmaceutically acceptable salt thereof, such as oseltamivir, zanamivir, peramivir, baloxavir and laninamivir.

In addition, the anti-viral agent according to the present invention may be a therapeutic agent for Herpes virus in the treatment of HSV (Herpes Simplex Virus), and VZV (Varicella Zoster Virus) infections, and the therapeutic agent for Herpes virus may be selected from the group consisting of aciclovir, valaciclovir, idoxuridine, vidarabine, penciclovir, famciclovir, trifluridine, cidofovir and foscarnet.

In addition, the anti-viral agent according to the present invention may be a therapeutic agent for Hepatitis B virus, which inhibits the replication of hepatitis B virus, thereby preventing inflammation, fibrosis, cirrhosis, and hepatocellular carcinoma, and the agent may be selected from the group consisting of lamivudine, clevudine, telbivudine, entecavir, adefovir, tenofovir disoproxil, tenofovir alafenamide, and besifovir. When resistance develops, it may be changed another agent or combination treatment of the two drugs.

In addition, the anti-viral agent according to the present invention may be a therapeutic agent for Hepatitis C virus that delays the progression of the disease by inhibiting the proliferation of the hepatitis C virus, and the agent may be peginterferon-α-2a and peginterferon-α-2b, protease family compound, or its derivative, or a pharmaceutically acceptable salt therof, such as ribavirin, or antiviral agent acting on viral proteins, such as DAA (Direct-acting antivirals) family compound, or its derivative, or a pharmaceutically acceptable salt therof, and it may be selected from the group consisting of boceprevir, dasabuvir, daclatasvir, asunaprevir, sofosbuvir as monotherapy, and elbasvir/grazoprevir, glecaprevir/pibrentasvir, ombitasvir/paritaprevir/ritonavir as combination therapy.

In addition, the anti-viral agent according to the present invention may be immune enhancer (Interferones) family compound, or its derivative, or a pharmaceutically acceptable salt thereof, and the immune enhancer may be selected from the group consisting of interferon alfa-2a, interferon alfa-2b and peginterferon-α-2a.

The anti-viral agent according to the present invention may be immune modulator family compound, or its derivative, or a pharmaceutically acceptable salt thereof, and the immune modulator may be selected from imiquimod.

In addition, the antidepressant according to the present invention may be TCAs (Tricyclic antidepressants), and the TCAs may be selected from the group consisting of amitriptyline, clomipramine, nortriptyline, desipramine, imipramine, amoxapine, maprotiline, trimipramine, protriptyline and melitracen.

In addition, the antidepressant according to the present invention may be SSRIs (Selective serotonin reuptake inhibitors), and the SSRIs may be selected from the group consisting of fluoxetine, fluvoxamine, paroxetine, sertraline, escitalopram and vortioxetine.

In addition, the antidepressant according to the present invention may be MAOI (Monoamine oxidase inhibitor) and the MAOI may be moclobemide.

In addition, the antidepressant according to the present invention may be SNRIs (Serotonin-norepinephrine reuptake inhibitors), and the SNRIs may be selected from the group consisting of duloxetine, venlafaxine, desbenlafaxine, milnacipran, bupropion, mirtazapine, trazodone, and tianeptine.

In addition, the biguanide-based compound according to the present invention may be selected from the group consisting of metformin, phenformin, buformin and biguanide.

In the present invention, metformin is a compound represented by the following Chemical Formula 1:

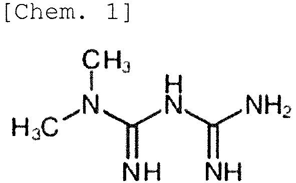

In the present invention, phenformin is a compound represented by the following Chemical Formula 2:

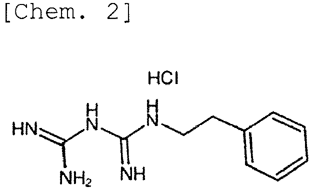

In the present invention, buformin is a compound represented by the following Chemical Formula 3:

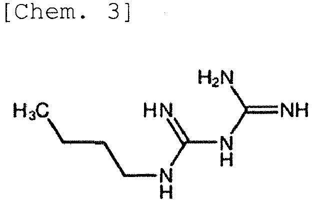

In the present invention, biguanide is a compound represented by the following Chemical Formula 4:

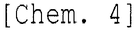

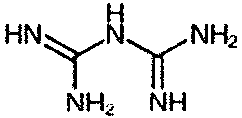

In an aspect of the present invention, the concentration of the anti-viral agent may be from 0.001 µM to 10 mM, and the concentration of the antidepressant may be from 0.001 µM to 10 mM.

In an aspect of the present invention, the concentration of the biguanide-based compound, or a pharmaceutically acceptable salt thereof that may be further added, may be from 0.1 mM to 100 mM.

In an aspect of the present invention, the contents of the anti-viral agnet; antidepressant; and biguanide-based compound or a pharmaceutically acceptable salt thereof in the medicament of the present invention may be appropriately selected depending on the form of the preparation, and the like.

In an aspect of the present invention, in a case where the anti-viral agent; antidepressant; and biguanide-based compound or a pharmaceutically acceptable salt thereof are formulated into a single preparation, the content of the anti-viral agent is generally from about 0.01 to about 99.99 wt%, specifically from about 0.01 to about 90 wt%, preferably from about 0.1 to about 90 wt%, more preferably from about 0.1 to about 80 wt %, still more preferably from about 0.1 to about 70 wt % with respect to the entire preparation, and the content of the antidepressant is generally from about 0.01 to about 99.99 wt%, specifically from about 0.01 to about 90 wt%, preferably from about 0.1 to about 80 wt%, more preferably from about 0.1 to about 70 wt %, and still more preferably from about 0.1 to about 60 wt % with respect to the entire preparation, and the content of the biguanide-based compound or a pharmaceutically acceptable salt thereof is generally from about 0.01 to about 99.99 wt%, specifically from about 0.01 to about 90 wt%, preferably from about 0.1 to about 90 wt%, more preferably from about 0.1 to about 80 wt %, still more preferably from about 0.1 to about 70 wt % with respect to the entire preparation.

Meanwhile, in the case of being blended as a single preparation, the anti-viral agent; and antidepressant may be blended in the medicament of the present invention at a weight ratio of 0.0000001 to 10 parts by weight of a first component, anti-viral agent and 1 part by weight of a second component, antidepressant. In addition, in the case of being blended as a single preparation, the first component of the anti-viral agent; the second component of the antidepressant; and the third component of the biguanide-based compound or a pharmaceutically acceptable salt thereof may be blended in the medicament of the present invention at a weight ratio of 0.0000001 to 10 parts by weight of a first component : 1 part by weight of a second component : 0.01 to 100000 parts by weight of a third component.

In an aspect of the present invention, in a case where the anti-viral agent; the antidepressant; and the biguanide-based compound or a pharmaceutically acceptable salt thereof are formulated separately and used in combination, the content of the anti-viral agent in the preparation containing the anti-viral agent is generally from about 0.01 to about 99.99 wt%, specifically from about 0.1 to 99.99 wt%, preferably from about 0.1 to about 90 wt%, more preferably from about 0.1 to about 80 wt%, and still more preferably from about 1 to about 80 wt% with respect to the preparation containing the corresponding ingredient. In addition, the content of the antidepressant in the preparation containing the antidepressant is generally from about 0.01 to about 99.99 wt%, specifically from about 0.1 to 99.99 wt%, preferably from about 0.1 to about 90 wt%, more preferably from about 0.1 to about 80 wt%, and still more preferably from about 1 to about 80 wt% with respect to the preparation containing the corresponding ingredient. In addition, the content of the buguanide-based compound or a pharmaceutically acceptable salt thereof in the preparation containing the biguanide-based compound or a pharmaceutically acceptable salt thereof is generally from about 0.01 to about 99.99 wt%, specifically from about 0.1 to 99.99 wt%, preferably from about 0.1 to about 90 wt%, more preferably from about 0.1 to about 80 wt%, and still more preferably from about 1 to about 80 wt% with respect to the preparation containing the corresponding ingredient.

Meanwhile, in a case where the anti-viral agent; antidepressant; and biguanide-based compound or a pharmaceutically acceptable salt thereof are formulated separately and used in combination, the content of additives such as carriers is variable, but may be generally from about 1 to 99.00 wt %, specifically from about 1 to about 90 wt%, preferably from about 10 to about 90 wt%, more preferably from about 10 to 80 wt%, and still more preferably from about 10 to about 70 wt% with respect to each preparation containing the corresponding ingredient.

In an aspect of the present invention, the cancer may be selected from the group consisting of (A) breast cancers including (1) ductal carcinoma including ductal carcinoma in situ (DCIS) (comedocarcinoma, cribriform, papillary, micropapillary), invasive ductal carcinoma (IDC), ductal carcinoma, mucinous (colloidal) carcinoma, papillary carcinoma, metaplastic carcinoma and inflammatory carcinoma; (2) lobular carcinomas, including lobular carcinoma in situ (LCIS) and invasive lobular carcinoma; and (3) Paget's disease of the nipple; (B) cancers of the female reproductive system, including (1) cancers of the cervix, including cervical intraepithelial neoplasia (grade I), cervical intraepithelial neoplasia (grade II), cervical intraepithelial neoplasia (grade III) (squamous cell carcinoma in situ), keratinizing squamous cell carcinoma, nonkeratinizing squamous cell carcinoma, verrucous carcinoma, adenocarcinoma in situ, adenocarcinoma in situ, endometrial type carcinoma, endometrioid adenocarcinoma, clear cell adenocarcinoma, adenoepithelioma, adenoid cystic carcinoma, small cell carcinoma and undifferentiated carcinoma; (2) cancers of the uterine body, including endometrioid carcinoma, adenocarcinoma, adenoacanthoma (adenocarcinoma with squamous metaplasia), adenoepithelioma (mixed adenocarcinoma and squamous cell carcinoma), mucinous adenocarcinoma, serous adenocarcinoma, clear cell adenocarcinoma, squamous cell adenocarcinoma and undifferentiated adenocarcinoma; (3) cancers of the ovary, including serous cystadenoma, serous cystadenoma, mucinous cystadenoma, mucinous cystadenoma, endometrioid tumor, endometrioid adenocarcinoma, clear cell tumor, clear cell cystadenoma and unclassified tumors; (4) cancers of the vagina, including squamous cell carcinoma and adenocarcinoma; and (5) vulvar cancers, including vulvar intraepithelial neoplasia (grade I), vulvar intraepithelial neoplasia (grade II), vulvar intraepithelial neoplasia (grade III) (squamous cell carcinoma in situ); squamous cell carcinoma, verrucous carcinoma, Paget's disease of the vulva, adenocarcinoma (NOS); basal cell carcinoma (NOS) and Bartholin gland carcinoma; (C) cancers of the male reproductive system, including (1) cancer of the penis, including squamous cell carcinoma; (2) cancers of the prostate, including adenocarcinomas, sarcomas, and transitional cell carcinomas of the prostate; and (3) cancers of the testes, including seminoma tumor, non-seminoma tumor, teratomas, embryonic carcinomas, yolk sac tumor and choriocarcinoma; (D) cancers of the heart system, including sarcomas (angiosarcoma, fibrosarcoma, rhabdomyosarcoma, liposarcoma), myxoma, rhabdomyoma, fibroma, lipoma and teratoma; (E) cancers of the respiratory system, including squamous cell carcinoma of the larynx, primary pleural mesothelioma and squamous cell carcinoma of the pharynx; (F) cancers of the lung, including squamous cell carcinoma (epidermoid carcinoma), a variant of squamous cell carcinoma, spindle cell carcinoma, small cell carcinoma, carcinoma of other cells, carcinoma of the intermediate cell type, complex oat cell carcinoma, adenocarcinoma, acinar adenocarcinoma, papillary adenocarcinoma, bronchoalveolar carcinoma, mucin-producing solid carcinoma, giant cell carcinoma, giant cell carcinoma, clear cell carcinoma and sarcoma; (G) cancers of the gastrointestinal tract, including (1) cancers of the ampulla of vater, including primary adenocarcinoma, carcinoid tumor and lymphoma; (2) cancers of the anal canal, including adenocarcinoma, squamous cell carcinoma and melanoma; (3) cancers of the extrahepatic bile duct, including carcinoma in situ, adenocarcinoma, papillary adenocarcinoma, adenocarcinoma, intestinal type, mucinous adenocarcinoma, clear cell adenocarcinoma, signet ring cell carcinoma, adenoepithelioma, squamous cell carcinoma, small cell (oat cell) carcinoma, undifferentiated carcinoma, carcinoma (NOS), sarcoma and carcinoid tumor; (4) cancers of the colon and rectum, including adenocarcinoma in situ, adenocarcinoma, mucinous adenocarcinoma (colloidal type; > 50% mucinous carcinoma), signet ring cell carcinoma (greater than 50% of signet ring cells), squamous cell (epidermoid) carcinoma, adenoepithelioma, small cell (oat cell) carcinoma, undifferentiated carcinoma, carcinoma (NOS), sarcoma, lymphoma and carcinoid tumor; (5) cancers of the esophagus, including squamous cell carcinoma, adenocarcinoma, leiomyosarcoma and lymphoma; (6) cancers of the gallbladder, including adenocarcinoma, adenocarcinoma, bowel type, adenoepithelioma, carcinoma in situ, carcinoma (NOS), clear cell adenocarcinoma, mucinous adenocarcinoma, papillary adenocarcinoma, signet ring cell carcinoma, small cell (oat cell) carcinoma, squamous cell carcinoma and undifferentiated carcinoma; (7) cancers of the lips and oral cavity, including squamous cell carcinoma; (8) cancers of the liver, including liver cancer (hepatocellular carcinoma), cholangiocarcinoma, hepatoblastoma, hemangiosarcoma, hepatocellular adenoma and hemangioma; (9) cancers of the exocrine pancreas, including ductal cell carcinoma, giant cell carcinoma multiforme, giant cell carcinoma, osteoclastoid type, adenocarcinoma, adenoepithelioma, mucinous (colloidal) carcinoma, cystadenoma, acinar cell carcinoma, papillary carcinoma, small cell (oat cell) carcinoma, mixed cell type, carcinoma (NOS), undifferentiated carcinoma, endocrine cell tumor arising from islet cells of Langerhans and carcinoid tumor; (10) cancers of the salivary glands, including acinar (acinar) cell carcinoma, adenoid cystic carcinoma (cylindroma), adenocarcinoma, squamous cell carcinoma, carcinoma in pleomorphic adenoma (malignant mixed tumor), mucoepidermoid carcinoma (well-differentiated or low grade) and mucoepidermoid carcinoma (poorly differentiated or high grade); (11) cancers of the stomach, including adenocarcinoma, papillary adenocarcinoma, tubular adenocarcinoma, mucinous adenocarcinoma, signet ring cell carcinoma, adenoepithelioma, squamous cell carcinoma, small cell carcinoma, undifferentiated carcinoma, lymphoma, sarcoma and carcinoid tumor; and (12) cancers of the small intestine, including adenocarcinoma, lymphoma, carcinoid tumor, Kaposi's sarcoma, leiomyoma, hemangioma, lipoma, neurofibromatosis and fibroma; (H) cancers of the urinary system, including (1) cancers of the kidney, including renal cell carcinoma, carcinoma of the Bellini collecting duct, adenocarcinoma, papillary carcinoma, tubular carcinoma, granular cell tumor, clear cell carcinoma (adenocarcinoma of the kidney), sarcoma of the kidney and nephroblastoma; (2) cancers of the renal pelvis and ureter, including transitional cell carcinoma, papillary transitional cell carcinoma, squamous cell carcinoma and adenocarcinoma; (3) cancers of the urethra, including transitional cell carcinoma, squamous cell carcinoma and adenocarcinoma; and (4) cancers of the bladder, including carcinoma in situ, transitional urothelial cell carcinoma, papillary transitional cell carcinoma, squamous cell carcinoma, adenocarcinoma, and undifferentiated carcinoma; and(I) cancers of muscles, bones and soft tissues, including (1) cancers of the bone, including (a) osteogenesis: osteosarcoma; (b) chondrogenesis: chondrosarcoma and mesenchymal chondrosarcoma; (c) giant cell tumor, malignant; (d) Ewing's sarcoma; (e) vascular tumors: hemangioendothelioma, hemangiopericytoma and hemangiosarcoma; (f) connective tissue tumors: fibrosarcoma, liposarcoma, malignant mesenchymoma and undifferentiated sarcoma; and (g) other tumors: chordoma and adamantinoma of the long bones; (2) cancers of soft tissues, including alveolar soft part sarcoma, angiosarcoma, epithelioid sarcoma, extraosseous chondrosarcoma, fibrosarcoma, leiomyosarcoma, liposarcoma, malignant fibrous histiocytoma, malignant hemangiopericytoma, malignant mesenchymoma, malignant Schwannoma, rhabdomyosarcoma, synovial sarcoma and sarcoma (NOS); (3) cancers of the nervous system, including cancers of the skull (osteoma, hemangioma, granuloma, xanthoma, and osteitis deformans), cancers of the meninges (meningioma, meningiosarcoma, and gliomatosis), cancers of the brain (astrocytoma, meduloblastoma, glioma, ependymal glioma, germinoma (pineal tumor), glioblastoma multiforme, oligodendrocytoma, schwannoma, retinoblastoma, and congenital tumor), and cancers of the spinal cord (neurofibromatosis, meningioma, glioma, sarcoma); (4) hematologic malignancies, including myeloid leukemia (acute and chronic), acute lymphoblastic leukemia, chronic lymphocytic leukemia, myeloproliferative disease, multiple myeloma; myelodysplastic syndrome, Hodgkin's disease and non-Hodgkin's lymphoma (malignant lymphoma); (5) cancers of the endocrine system, including (a) cancers of the thyroid gland, including papillary carcinoma (including those of the follicular region), follicular carcinoma, medullary carcinoma and undifferentiated (anaplastic) carcinoma; and (b) neuroblastomas, including sympathicogonioma, sympathoblastoma, malignant ganglioneuroma, ganglioneuroblastoma and ganglioneuroma; (6) cancers of the skin, including squamous cell carcinoma, spindle cell squamous cell carcinoma, basal cell carcinoma, adenocarcinoma arising from sweat glands or sebaceous glands, and malignant melanoma; and (7) cancers of the eye, including (a) cancer of the conjunctiva, including carcinoma of the conjunctiva; (b) cancers of the eyelid, including basal cell carcinoma, squamous cell carcinoma, melanoma of the eyelid and sebaceous cell carcinoma; (c) cancers of the lacrimal gland, including adenocarcinoma, adenoid cystic carcinoma, carcinoma in pleomorphic adenoma, mucoepidermoid carcinoma and squamous cell carcinoma; (d) cancers of the uvea, including spindle cell melanoma, mixed cell melanoma and epithelioid cell melanoma; (e) cancers of the orbit, including sarcoma of the orbit, soft tissue tumor, and sarcoma of the bone; and (f) retinoblastoma.

In an aspect of the present invention, the pharmaceutical composition may be formulated into a formulation selected from the group consisting of a tablet, a capsule, an injection, a troche, a powder, a granule, a solution, a suspension, an oral solution, an emulsion, a syrup, a suppository, a vaginal tablet and a pill, but is not limited thereto, and can be formulated in an appropriate formulation as needed.

In addition, the present invention provides a combination kit for preventing or treating cancer comprising complexed, mixed or combined preparation of an anti-viral agent; and an antidepressant.

In addition, the present invention provides a combination kit for preventing or treating cancer, comprising the complex, mixed or combined preparation, and the kit comprising an anti-viral agent and an antidepressant, and a biguanide-based compound or a pharmaceutically acceptable salt thereof that may be added.

In an aspect of the present invention, an anti-viral agent, an antidepressant, and a biguanide-based compound or a pharmaceutically acceptable salt thereof, the content and content ratio of the component, and the cancer are the same as those in the description of the pharmaceutical composition for the prevention or treatment of cancer, and specific description thereof refers to the above contents.

In the present invention, an anti-viral agent, an antidepressant and/or biguanide-based compound or its derivative may be used in the form of a pharmaceutically acceptable salt, and an acid addition salt formed using a pharmaceutically acceptable free acid is useful as the salt. Acid addition salts are obtained from inorganic acids such as hydrochloric acid, nitric acid, phosphoric acid, sulfuric acid, hydrobromic acid, hydroiodic acid, nitrous acid or phosphorous acid and non-toxic organic acids such as aliphatic mono and dicarboxylates, phenyl-substituted alkanoates, hydroxy alkanoates and alkandioates, aromatic acids, and aliphatic and aromatic sulfonic acids. Such pharmaceutically non-toxic salts include sulfates, pyrosulfates, bisulfates, sulfites, bisulfites, nitrates, phosphates, monohydrogen phosphates, dihydrogen phosphates, metaphosphates, pyrophosphate chloride, bromides, iodides, fluorides, acetates, propionates, decanoates, caprylates, acrylates, formates, isobutyrates, caprates, heptanoates, propiolates, oxalates, malonates, succinates, suberates, sebacates, fumarates, maleates, butyne-1,4-dioates, hexane-1,6-dioates, benzoates, chlorobenzoates, methylbenzoates, dinitrobenzoates, hydroxybenzoates, methoxybenzoates, phthalates, terephthalates, benzenesulfonates, toluenesulfonates, chlorobenzenesulfonates, xylenesulfonates, phenylacetates, phenylpropionates, phenylbutyrates, citrates, lactates, hydroxybutyrates, glycolates, malates, tartrates, methanesulfonates, propanesulfonates, naphthalene-1-sulfonates, naphthalene-2-sulfonates or mandelates.

The acid addition salt according to the present invention may be produced by a conventional method, for example, by dissolving the compound according to the present invention in an excess aqueous acid solution and precipitating this salt using a water-miscible organic solvent, for example, methanol, ethanol, acetone or acetonitrile. The acid addition salt may also be produced by evaporating the solvent or excess acid from this mixture to dryness, or by performing suction filtration of the precipitated salt.

In addition, a pharmaceutically acceptable metal salt may be produced using a base. An alkali metal or alkaline earth metal salt is obtained, for example, by dissolving the compound in an excess alkali metal hydroxide or alkaline earth metal hydroxide solution, filtering the undissolved compound salt, and evaporating and drying the filtrate. In this case, it is pharmaceutically suitable to prepare a sodium, potassium or calcium salt as the metal salt. A silver salt corresponding thereto is obtained by reacting the alkali metal or alkaline earth metal salt with a suitable silver salt (for example, silver nitrate).

In the case of formulating the composition into a preparation, the preparation is usually manufactured using commonly used diluents or excipients such as fillers, extenders, binders, wetting agents, disintegrants, and surfactants.

A solid preparation for oral administration includes tablets, pills, powders, granules, capsules, troches and the like, and such a solid preparation is prepared by mixing one or more compounds of the present invention with at least one or more excipients, for example, starch, calcium carbonate, sucrose or lactose or gelatin. In addition to simple excipients, lubricants such as magnesium stearate and talc are also used. Liquid preparations for oral administration include suspensions, oral solutions, emulsions, or syrups, and may contain various excipients, for example, wetting agents, sweetening agents, fragrances, and preservatives in addition to water and liquid paraffin, which are commonly used simple diluents.

Preparations for parenteral administration contain sterile aqueous solutions, non-aqueous solvents, suspension solvents, emulsions, lyophilized preparations, suppositories, and the like.

As non-aqueous solvents and suspension solvents, propylene glycol, polyethylene glycol, vegetable oils such as olive oil, injectable esters such as ethyl oleate, and the like may be used. As the base of suppositories, witepsol, macrogol, tween 61, cacao butter, laurin oil, glycerol, gelatin, and the like may be used.

In the present invention, the "pharmaceutically effective amount" refers to an amount sufficient to treat a disease at an effective benefit/risk ratio applicable to medical treatment, and the effective dose level may be determined depending on factors including the kind of patient's disease, severity, drug activity, drug sensitivity, administration time, administration route and excretion rate, treatment period, and concomitant drugs and other factors well known in the medical arts. The composition of the present invention may be administered as a blended individual therapeutic agent or may be administered with other therapeutic agents in a complex, mixed or combined manner, may be administered sequentially or simultaneously with conventional therapeutic agents, and may be administered single time or multiple times. It is important to administer the composition in a minimum amount to obtain the maximum effect without side effects in consideration of all of the factors mentioned above, and the minimum amount may be easily determined by those skilled in the art.

Specifically, the effective amount of the compound according to the present invention may vary depending on the age, sex, and weight of the patient, and the compound may be administered by generally from 0.1 mg to 100 mg, preferably from 0.5 mg to 10 mg per 1 kg of body weight daily or every other day or in divided doses 1 to 3 times a day. However, the dosage is not intended to limit the scope of the present invention in any way since the effective amount may increase or decrease depending on administration route, severity of disease, sex, weight, age and the like.

In an aspect of the present invention, a first component including an anti-viral agent; and a second component including an antidepressant are administered to a subject in need of cancer treatment in a blended, complex, mixed or combined manner. In addition, in an aspect of the present invention, a first component including an anti-viral agent; a second component including antidepressant; and a third component including a biguanide-based compound, or a pharmaceutically acceptable salt thereof may be further added as an active ingredient and are administered to a subject in need of cancer treatment in a blended, complex, mixed or combined manner. Various cancers, including the above-mentioned cancer diseases, can be treated by the method according to the present invention.

In a specific example of the present invention, the inventors confirmed the anti-cancer activity of a complex, mixed, or combination preparation of an antiviral agent and an antidepressant by treating pancreatic and colon cancer cells with NNRTI antiviral agents including efavirenz, etravirine, rilpivirine, and PI agent lopinavir, as well as SSRI antidepressants including fluoxetine, fluvoxamine, paroxetine, sertraline, SNRI including duloxetine, and TCA antidepressants including amitriptyline, clomipramine, nortriptyline, desipramine, amoxapine, maprotiline, trimipramine, protriptyline, and melitracen alone or in a complex, mixed, or combination treatment, and performing MTT analysis, thereby confirming growth inhibitory effects in cancer cells.

In another specific example of the present invention, the inventors confirmed the anti-cancer activity of a complex, mixed, or combination preparation of an antiviral agent, an antidepressant, and a biguanide compound or its pharmaceutically acceptable salt by treating pancreatic and colon cancer cells with NNRTI antiviral agent efavirenz, SSRI antidepressant fluoxetine, and biguanide compound metformin alone or in a complex, mixed, or combination treatment, and performing MTT analysis, thereby confirming growth inhibitory effects in cancer cells.

Furthermore, in a specific embodiment of the present invention, the inventors confirmed the anti-cancer activity of antiviral agents and antidepressants; and antiviral agents, antidepressants, and biguanide compounds or pharmaceutically acceptable salts thereof, by treating colon cancer cells with antiviral agent NNRTIs such as efavirenz, antidepressant SSRIs such as fluoxetine, and biguanide compound metformin alone or in combination, mixture or co-treatment for 0, 24, 48, 72 and 96 hours and performing MTT analysis, showing growth inhibition effects in cancer cells. In addition, it was confirmed that the growth inhibition effect was superior when efavirenz, fluoxetine, and metformin were co-treated than when efavirenz and fluoxetine, efavirenz and metformin, or fluoxetine and metformin were co-treated.

In addition, it has been confirmed that the synergistic effect showing significantly higher growth inhibition is exerted in a case where the cancer cells are treated with an anti-viral agent, antidepressant, and biguanide-based compound in a complex, mixed, or combined manner compared to a case where the cancer cells are treated with each of these singly.

In the present invention, the subject is a mammal in need of cancer treatment. Typically, the subject is a human cancer patient. In an aspect of the present invention, the subject may be a non-human mammal, such as a non-human primate, an animal used in the model system (for example, a mouse and a rat used for screening, characterization, and evaluation of pharmaceuticals), and other mammals, for example a primate animal such as a rabbit, guinea pig, hamster, dog, cat, chimpanzee, gorilla, or monkey.

In an aspect of the present invention, the pharmaceutical composition may be used singly or concurrently with surgery, hormone therapy, drug therapy, and biological response modifiers for the treatment of cancer patients.

The present invention also provides use of a complex, mixed or combined preparation of an anti-viral agent; and antidepressant to be used as a pharmaceutical composition for prevention or treatment of cancer.

In addition, the present invention also provides use as a health food for preventing and improving cancer, comprising a complex, mixed or combined preparation of an anti-viral agent, and antidepressant or a pharmaceutically acceptable salt thereof.

### [Examples]

Hereafter, the present invention will be described in more detail through Examples and Experimental Examples. However, the following Examples and Experimental Examples are intended to help the understanding of the present invention but are not intended to limit the scope of the present invention.

### <Example 1> Confirmation of anticancer activity of an anti-viral agent NNRTIs(Non-nucleoside reverse-transcriptase inhibitors) and an antidepressant SSRIs (Selective serotonin reuptake inhibitors)

### <1-1> Confirmation of anticancer activity of efavirenz and fluoxetine in pancreatic and colon cancers

To investigate the anticancer activity of an anti-viral agent NNRTI and an antidepressant SSRI, pancreatic cancer cells and colon cancer cells were treated with efavirenz as an anti-viral agent NNRTI, and fluoxetine as an antidepressant SSRI and MTT (3-(4,5-dimethylthiazol-2-yl)-2,5-diphenyltetrazolium bromide) analysis was conducted to examine growth inhibition.

Specifically, ASPC-1 cell line, which is a pancreatic cancer cell line, and HCT116 cell line, which is a colon cancer cell line, was incubated in a 100 mm culture dish using DMEM-10% FBS under 5% CO₂ at 37°C, inoculated into each well of a 96-well plate at 20% confluence, and incubated for 24 hours. The incubated cell lines were treated with efavirenz at a concentration of 2 µM and fluoxetine at a concentration of 2 µM alone or in combination and cultured in a CO₂ incubator for 24 hours. The culture medium was removed from each well, 100 µl of a fresh culture medium was added, and 10 µl of a 12 mM MTT stock solution (5 mg MTT/PBS) was added, and incubation was performed at 37°C for 2 hours. Thereafter, 100 µl of SDS-HCl solution (1 g SDS/10 ml 0.01 M HCl), which was a reaction terminating solution, was added, incubation was performed at 37°C for 4 hours, and OD was measured at 570 nM using a microplate leader. Percentage (%) growth inhibition was calculated by comparing the OD thus measured to the OD of the cells not treated with drugs.

As a result, as illustrated in FIG. 1, it has been confirmed that a ceiling effect showing significantly higher growth inhibition is exerted in an all the case when pancreatic cancer cells and colon cancer cells were treated with 2 µM efavirenz and 2 µM fluoxetine in combination than when 2 µM efavirenz and 2 µM fluoxetine were treated alone (FIG. 1).

### <1-2> Confirmation of anticancer activity of efavirenz and fluvoxamine in pancreatic and colon cancers

To investigate the anticancer activity of an anti-viral agent NNRTI and an antidepressant SSRI, pancreatic cancer cells and colon cancer cells were treated with efavirenz as an anti-viral agent NNRTI, and fluvoxamine as an antidepressant SSRI and MTT analysis was conducted to examine growth inhibition.

Specifically, MTT analysis was performed in the same manner as described above Example <1-1> using a pancreatic cancer cell line, and a colon cancer cell line. The drug was treated and cultured for 24 hours.

As a result, as illustrated in FIG 2, it has been confirmed that a ceiling effect showing significantly higher growth inhibition is exerted when pancreatic cancer cells and colon cancer cells were treated with 2 µM efavirenz and 10 µM fluvoxamine in combination than when 2 µM efavirenz and 10 µM fluvoxamine were treated alone (FIG. 2).

### <1-3> Confirmation of anticancer activity of efavirenz and paroxetine in pancreatic and colon cancers

To investigate the anticancer activity of an anti-viral agent NNRTI and an antidepressant SSRI, pancreatic cancer cells and colon cancer cells were treated with efavirenz as an anti-viral agent NNRTI, and paroxetine as an antidepressant SSRI and MTT analysis was conducted to examine growth inhibition.

Specifically, MTT analysis was performed in the same manner as described above Example <1-1> using a pancreatic cancer cell line, and a colon cancer cell line. The drug was treated and cultured for 24 hours.

As a result, as illustrated in FIG 3, it has been confirmed that a ceiling effect showing significantly higher growth inhibition is exerted when pancreatic cancer cells and colon cancer cells were treated with 2 µM efavirenz and 1 µM paroxetine in combination than when 2 µM efavirenz and 1 µM paroxetine were treated alone (FIG. 3).

### <1-4> Confirmation of anticancer activity of efavirenz and sertraline in pancreatic and colon cancers

To investigate the anticancer activity of an anti-viral agent NNRTI and an antidepressant SSRI, various cancer cells were treated with efavirenz as an anti-viral agent NNRTI, and sertraline as an antidepressant SSRI and MTT analysis was conducted to examine growth inhibition.

Specifically, MTT analysis was performed in the same manner as described above Example <1-1> using a pancreatic cancer cell line, and a colon cancer cell line. The drug was treated and cultured for 24 hours.

As a result, as illustrated in FIG. 4, it has been confirmed that a ceiling effect showing significantly higher growth inhibition is exerted when pancreatic cancer cells and colon cancer cells were treated with 2 µM efavirenz and 1 µM sertraline in combination than when 2 µM efavirenz and 1 µM sertraline were treated alone (FIG. 4).

### <1-5> Confirmation of anticancer activity of etravirine and fluoxetine in pancreatic and colon cancers

To investigate the anticancer activity of an anti-viral agent NNRTI and an antidepressant SSRI, pancreatic cancer cells and colon cancer cells were treated with etravirine as an anti-viral agent NNRTI, and fluoxetine as an antidepressant SSRI and MTT analysis was conducted to examine growth inhibition.

Specifically, MTT analysis was performed in the same manner as described above Example <1-1> using a pancreatic cancer cell line, and a colon cancer cell line. The drug was treated and cultured for 24 hours.

As a result, as illustrated in FIG 5, it has been confirmed that a ceiling effect showing significantly higher growth inhibition is exerted when pancreatic cancer cells and colon cancer cells were treated with 10 µM etravirine and 2 µM fluoxetine in combination than when 10 µM etravirine and 2 µM fluoxetine were treated alone (FIG. 5).

### <1-6> Confirmation of anticancer activity of rilpivirine and fluoxetine in pancreatic and colon cancers

To investigate the anticancer activity of an anti-viral agent NNRTI and an antidepressant SSRI, colon cancer cells were treated with rilpivirine as an anti-viral agent NNRTI, and fluoxetine as an antidepressant SSRI and MTT analysis was conducted to examine growth inhibition.

Specifically, MTT analysis was performed in the same manner as described above Example <1-1> using a pancreatic cancer cell line, and a colon cancer cell line. The drug was treated and cultured for 24 hours.

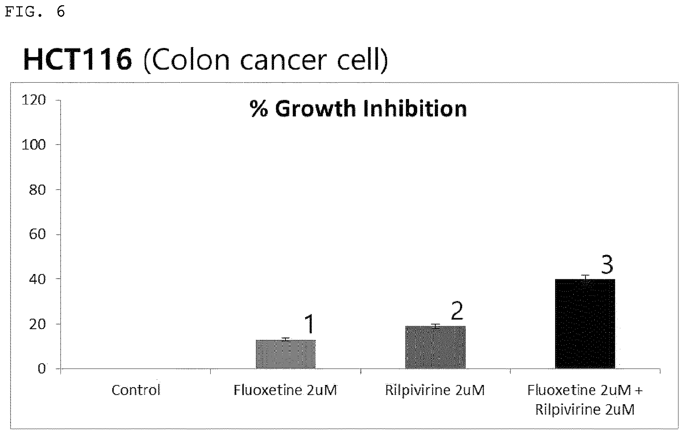

As a result, as illustrated in FIG 6, it has been confirmed that a ceiling effect showing significantly higher growth inhibition is exerted when colon cancer cells were treated with 2 µM rilpivirine and 2 µM fluoxetine in combination than when 2 µM rilpivirine and 2 µM fluoxetine were treated alone (FIG. 6).

### <Example 2> Confirmation of anticancer activity of anti-viral agent NNRTIs(Non-nucleoside reverse-transcriptase inhibitors) and antidepressant SNRIs(Serotonin-norepinephrine reuptake inhibitors)

### <2-1> Confirmation of anticancer activity of efavirenz and duloxetine in pancreatic and colon cancers

To investigate the anticancer activity of an anti-viral agent NNRTI and an antidepressant SNRI, pancreatic cancer cells and colon cancer cells were treated with efavirenz as an anti-viral agent NNRTI, and duloxetine as an antidepressant SNRI and MTT analysis was conducted to examine growth inhibition.

Specifically, MTT analysis was performed in the same manner as described above Example <1-1> using a pancreatic cancer cell line, and a colon cancer cell line. The drug was treated and cultured for 24 hours.

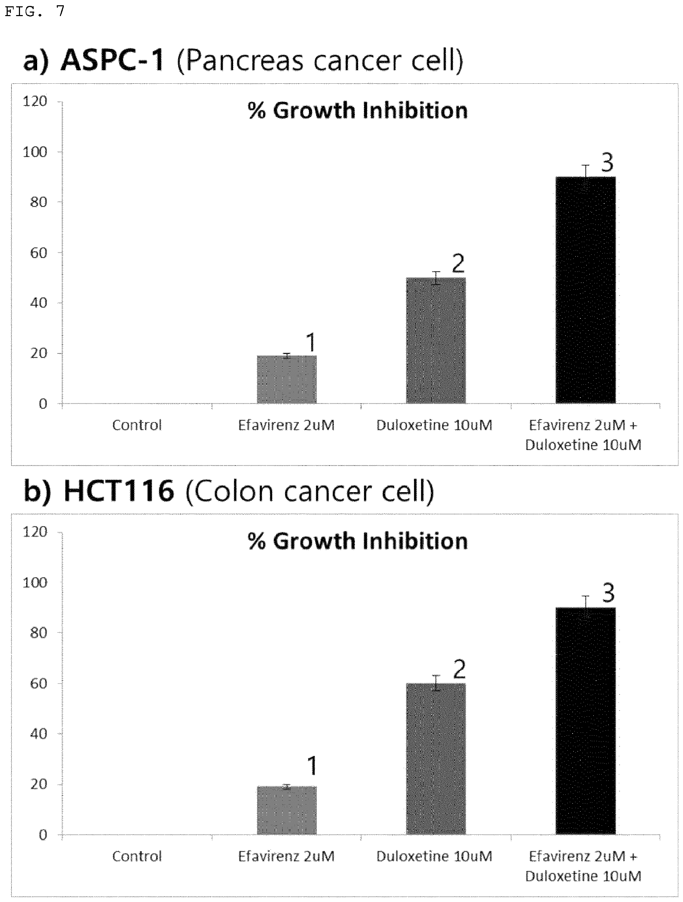

As a result, as illustrated in FIG 7, it has been confirmed that a ceiling effect showing significantly higher growth inhibition is exerted when pancreatic cancer cells and colon cancer cells were treated with 2 µM efavirenz and 10 µM duloxetine in combination than when 2 µM efavirenz and 10 µM duloxetine were treated alone (FIG. 7).

### <Example 3> Confirmation of anticancer activity of anti-viral agent NNRTIs(Non-nucleoside reverse-transcriptase inhibitors) and antidepressant TCAs(Tricyclic antidepressants)

### <3-1> Confirmation of anticancer activity of efavirenz and amitriptyline in pancreatic and colon cancers

To investigate the anticancer activity of an anti-viral agent NNRTI and an antidepressant TCA, pancreatic cancer cells and colon cancer cells were treated with efavirenz as an anti-viral agent NNRTI, and amitriptyline as an antidepressant TCA and MTT analysis was conducted to examine growth inhibition.

Specifically, MTT analysis was performed in the same manner as described above Example <1-1> using a pancreatic cancer cell line, and a colon cancer cell line. The drug was treated and cultured for 24 hours.

As a result, as illustrated in FIG 8, it has been confirmed that a ceiling effect showing significantly higher growth inhibition is exerted when pancreatic cancer cells and colon cancer cells were treated with 2 µM efavirenz and 10 µM amitriptyline in combination than when 2 µM efavirenz and 10 µM amitriptyline were treated alone (FIG. 8).

### <3-2> Confirmation of anticancer activity of efavirenz and clomipramine in pancreatic and colon cancers

To investigate the anticancer activity of an anti-viral agent NNRTI and an antidepressant TCA, pancreatic cancer cells and colon cancer cells were treated with efavirenz as an anti-viral agent NNRTI, and clomipramine as an antidepressant TCA and MTT analysis was conducted to examine growth inhibition.

Specifically, MTT analysis was performed in the same manner as described above Example <1-1> using a pancreatic cancer cell line, and a colon cancer cell line. The drug was treated and cultured for 24 hours.

As a result, as illustrated in FIG 9, it has been confirmed that a ceiling effect showing significantly higher growth inhibition is exerted when pancreatic cancer cells and colon cancer cells were treated with 2 µM efavirenz and 10 µM clomipramine in combination than when 2 µM efavirenz and 10 µM clomipramine were treated alone (FIG. 9).

### <3-3> Confirmation of anticancer activity of efavirenz and nortriptyline in pancreatic and colon cancers

To investigate the anticancer activity of an anti-viral agent NNRTI and an antidepressant TCA, pancreatic cancer cells and colon cancer cells were treated with efavirenz as an anti-viral agent NNRTI, and nortriptyline as an antidepressant TCA and MTT analysis was conducted to examine growth inhibition.

Specifically, MTT analysis was performed in the same manner as described above Example <1-1> using a pancreatic cancer cell line, and a colon cancer cell line. The drug was treated and cultured for 24 hours.

As a result, as illustrated in FIG 10, it has been confirmed that a ceiling effect showing significantly higher growth inhibition is exerted when pancreatic cancer cells and colon cancer cells were treated with 2 µM efavirenz and 10 µM nortriptylin in combination than when 2 µM efavirenz and 10 µM nortriptylin were treated alone (FIG. 10).

### <3-4> Confirmation of anticancer activity of efavirenz and desipramine in pancreatic and colon cancers

To investigate the anticancer activity of an anti-viral agent NNRTI and an antidepressant TCA, pancreatic cancer cells and colon cancer cells were treated with efavirenz as an anti-viral agent NNRTI, and desipramine as an antidepressant TCA and MTT analysis was conducted to examine growth inhibition.

Specifically, MTT analysis was performed in the same manner as described above Example <1-1> using a pancreatic cancer cell line, and a colon cancer cell line. The drug was treated and cultured for 24 hours.

As a result, as illustrated in FIG 11, it has been confirmed that a ceiling effect showing significantly higher growth inhibition is exerted when pancreatic cancer cells and colon cancer cells were treated with 2 µM efavirenz and 10 µM desipramine in combination than when 2 µM efavirenz and 10 µM desipramine were treated alone (FIG. 11).

### <3-5> Confirmation of anticancer activity of efavirenz and amoxapine in pancreatic and colon cancers

To investigate the anticancer activity of an anti-viral agent NNRTI and an antidepressant TCA, pancreatic cancer cells and colon cancer cells were treated with efavirenz as an anti-viral agent NNRTI, and amoxapine as an antidepressant TCA and MTT analysis was conducted to examine growth inhibition.

Specifically, MTT analysis was performed in the same manner as described above Example <1-1> using a pancreatic cancer cell line, and a colon cancer cell line. The drug was treated and cultured for 24 hours.

As a result, as illustrated in FIG 12, it has been confirmed that a ceiling effect showing significantly higher growth inhibition is exerted when pancreatic cancer cells and colon cancer cells were treated with 2 µM efavirenz and 10 µM amoxapine in combination than when 2 µM efavirenz and 10 µM amoxapine were treated alone (FIG. 12).

### <3-6> Confirmation of anticancer activity of efavirenz and maprotiline in pancreatic and colon cancers

To investigate the anticancer activity of an anti-viral agent NNRTI and an antidepressant TCA, pancreatic cancer cells and colon cancer cells were treated with efavirenz as an anti-viral agent NNRTI, and maprotiline as an antidepressant TCA and MTT analysis was conducted to examine growth inhibition.

Specifically, MTT analysis was performed in the same manner as described above Example <1-1> using a pancreatic cancer cell line, and a colon cancer cell line. The drug was treated and cultured for 24 hours.

As a result, as illustrated in FIG 13, it has been confirmed that a ceiling effect showing significantly higher growth inhibition is exerted when pancreatic cancer cells and colon cancer cells were treated with 2 µM efavirenz and 10 µM maprotiline in combination than when 2 µM efavirenz and 10 µM maprotiline were treated alone (FIG. 13).

### <3-7> Confirmation of anticancer activity of efavirenz and trimipramine in pancreatic and colon cancers

To investigate the anticancer activity of an anti-viral agent NNRTI and an antidepressant TCA, pancreatic cancer cells and colon cancer cells were treated with efavirenz as an anti-viral agent NNRTI, and trimipramine as an antidepressant TCA and MTT analysis was conducted to examine growth inhibition.

Specifically, MTT analysis was performed in the same manner as described above Example <1-1> using a pancreatic cancer cell line, and a colon cancer cell line. The drug was treated and cultured for 24 hours.

As a result, as illustrated in FIG 14, it has been confirmed that a ceiling effect showing significantly higher growth inhibition is exerted when pancreatic cancer cells and colon cancer cells were treated with 2 µM efavirenz and 10 µM trimipramine in combination than when 2 µM efavirenz and 10 µM trimipramine were treated alone (FIG. 14).

### <3-8> Confirmation of anticancer activity of efavirenz and protriptyline in pancreatic and colon cancers

To investigate the anticancer activity of an anti-viral agent NNRTI and an antidepressant TCA, pancreatic cancer cells and colon cancer cells were treated with efavirenz as an anti-viral agent NNRTI, and protriptyline as an antidepressant TCA and MTT analysis was conducted to examine growth inhibition.

Specifically, MTT analysis was performed in the same manner as described above Example <1-1> using a pancreatic cancer cell line, and a colon cancer cell line. The drug was treated and cultured for 24 hours.

As a result, as illustrated in FIG 15, it has been confirmed that a ceiling effect showing significantly higher growth inhibition is exerted when pancreatic cancer cells and colon cancer cells were treated with 2 µM efavirenz and 10 µM protriptyline in combination than when 2 µM efavirenz and 10 µM protriptyline were treated alone (FIG. 15).

### <3-9> Confirmation of anticancer activity of efavirenz and melitracen in pancreatic and colon cancers

To investigate the anticancer activity of an anti-viral agent NNRTI and an antidepressant TCA, pancreatic cancer cells and colon cancer cells were treated with efavirenz as an anti-viral agent NNRTI, and melitracen as an antidepressant TCA and MTT analysis was conducted to examine growth inhibition.

Specifically, MTT analysis was performed in the same manner as described above Example <1-1> using a pancreatic cancer cell line, and a colon cancer cell line. The drug was treated and cultured for 24 hours.

As a result, as illustrated in FIG 16, it has been confirmed that a ceiling effect showing significantly higher growth inhibition is exerted when pancreatic cancer cells and colon cancer cells were treated with 2 µM efavirenz and 1 µM melitracen in combination than when 2 µM efavirenz and 1 µM melitracen were treated alone (FIG. 16).

### <Example 4> Confirmation of anticancer activity of anti-viral agent PIs(Protease inhibitors) and antidepressant SSRI

### <4-1> Confirmation of anticancer activity of lopinavir and fluoxetine in pancreatic and colon cancers

To investigate the anticancer activity of an anti-viral agent PI and an antidepressant SSRI, pancreatic cancer cells and colon cancer cells were treated with lopinavir as an anti-viral agent PI, and fluoxetine as an antidepressant SSRI and MTT analysis was conducted to examine growth inhibition.

Specifically, MTT analysis was performed in the same manner as described above Example <1-1> using a pancreatic cancer cell line, and a colon cancer cell line. The drug was treated and cultured for 24 hours.

As a result, as illustrated in FIG 17, it has been confirmed that a ceiling effect showing significantly higher growth inhibition is exerted when pancreatic cancer cells and colon cancer cells were treated with 10 µM lopinavir and 2 µM fluoxetine in combination than when 10 µM lopinavir and 2 µM fluoxetine were treated alone (FIG. 17).

### <Example 5> Confirmation of anticancer activity of anti-viral agent NNRTIs(Non-nucleoside reverse-transcriptase inhibitor), antidepressant SSRIs and biguanide-based compounds

### <5-1> Confirmation of anticancer activity of efavirenz, fluoxetine, and metformin in pancreatic and colon cancers

To investigate the anticancer activity of antiviral NNRTI efavirenz, antidepressant SSRI fluoxetine, and biguanide compound metformin, which are known to possess antiviral, antidepressant, and hypoglycemic activities, respectively, pancreas cancer cells and colon cancer cells were treated with these compounds, and the growth inhibition effect was confirmed by performing MTT analysis.

Specifically, the pancreatic cancer cell line ASPC-1 and colon cancer cell lines HCT116 were cultured in 100 mm culture dishes using DMEM-10% FBS and incubated at 5% CO₂, 37°C until 20% confluence was reached. Then, the cells were seeded into each well of a 96-well plate and incubated for 24 hours. Efavirenz was treated at concentrations of 2 µM, fluoxetine at concentrations of 4 µM, and metformin at concentrations of 2.5 mM, either alone or in combination. The cells were then incubated for 24 hours in a CO₂ incubator. After removing the culture medium from each well, fresh culture medium 100 µl was added, followed by 10 µl of 12 mM MTT stock solution (5 mg MTT/PBS), and incubated at 37°C for 2 hours. Then, 100 µl of SDS-HCl solution (1 g SDS/10 ml 0.01 M HCl) was added to stop the reaction, and the plate was further incubated for 4 hours at 37°C. The optical density (OD) was measured at 570 nm using a microplate reader. The growth inhibition percentage was calculated by comparing the OD of drug-treated cells with that of untreated cells.

As a result, as shown in FIG 18, it was confirmed that the combination treatment of 2 µM efavirenz, 4 µM fluoxetine, and 2.5 mM metformin exhibited a significant synergistic growth inhibition effect compared to treatment with 2 µM efavirenz, 4 µM fluoxetine, or 2.5 mM metformin alone in pancreatic cancer cell line and colon cancer cell line (FIG 18).

### <5-2> Comparison of the antitumor activity between the combination of efavirenz and fluoxetine and the combination of efavirenz, fluoxetine and metformin in colon cancer

To investigate the difference in antitumor activity between the combination of antiviral and antidepressant agents and the combination of antiviral, antidepressant agents, and biguanide compounds, colon cancer cells were treated with the antiviral agent NNRTI, the antidepressant agent SSRI, fluoxetine, and metformin, a biguanide-based compound, and the growth inhibition effect was confirmed by MTT analysis.

Specifically, MTT analysis was performed in the same manner as described above Example <1-1> using a colon cancer cell line. The drug was treated and cultured for 24 hours.

As a result, it was confirmed that the combination treatment of 1.5 µM efavirenz and 2 µM fluoxetine; 1.5 µM efavirenz and 0.5 mM metformin; 2 µM fluoxetine and 0.5 mM metformin; and 1.5 µM efavirenz, 2 µM fluoxetine, and 0.5 mM metformin showed a significantly increased growth inhibition compared to the treatment with 0.5 mM metformin, 2 µM doxorubicin, 1.5 µM efavirenz, and 2 µM fluoxetine alone for 0, 24, 48, 72 and 96 hours, as shown in FIG. 19.

In addition, it was observed that the combination therapy of 1.5 µM efavirenz, 2 µM fluoxetine and 0.5 mM metformin showed a superior synergistic effect, which is a significant increase in growth inhibition compared to treatment with 1.5 µM efavirenz and 2 µM fluoxetine; 1.5 µM efavirenz and 0.5 mM metformin; or 2 µM fluoxetine and 0.5 mM metformin (FIG. 19).

In conclusion, the combined treatment of antiviral and antidepressant drugs showed significantly superior growth inhibition effects in cancer cells compared to their individual treatments. Furthermore, the combined treatment of antiviral, antidepressant, and biguanide-based compounds showed remarkably enhanced growth inhibition effects in cancer cells. Moreover, when three types of compounds were combined, showed significantly superior growth inhibition effects compared to their individual or dual treatments, that is a treatment of antiviral and antidepressant drugs, biguanide and antiviral drugs, or biguanide and antidepressant drugs.

### [Industrial applicability]

The present invention relates to a pharmaceutical composition for cancer prevention or treatment comprising antiviral and antidepressant agents as active ingredients, and the addition of biguanide-based compound, and more specifically an antiviral agent, an antidepressant and biguanide-based compound or a pharmaceutically acceptable salt thereof according to the present invention exhibit a synergistic and significant anticancer effect against various cancer types when they are treated in the form of a complex, mixture, or combined preparation while showing a slight anticancer effect when each of them is treated singly, and so the pharmaceutical composition comprising them as an active ingredient in the form of a complex, mixture, or combined preparation can be used effectively for preventing or treating cancer.

## Claims

1. A pharmaceutical composition for the prevention or treatment of cancer, comprising a first component including an antiviral agent; and a second component including an antidepressant as active ingredients in complex, mixed, or combined preparation.

2. A pharmaceutical composition for the prevention or treatment of cancer, comprising a first component including an antiviral agent; a second component including an antidepressant; and a third component including a biguanide-based compound or a pharmaceutically acceptable salt thereof in the manner of a complex, mixed, or combined preparation.

3. The pharmaceutical composition according to claim 1 or claim 2, wherein the antiviral agent is a Non-nucleoside reverse-transcriptase inhibitors (NNRTIs), Nucleoside reverse-transcriptase inhibitors (NRTIs), Protease inhibitors(PIs), Integrase Strand Transfer Inhibitors(INSTI), fusion inhibitors, CCR5(Chemokine (C-C motif) ligand 5) inhibitors, flu treatment agent, herpes treatment agent, hepatitis B treatment agent, hepatitis C treatment agent, immune enhancer, immune response modulator-based compound, its derivatives, or a pharmaceutically acceptable salt thereof.

4. The pharmaceutical composition according to claim 3, wherein the antiviral agent is one or more compound selected from the group consisting of efavirenz, etravirine, nevirapine, doravirine, rilpivirine, delavirdine, zidovudine, didanosine, zalcitabine, stavudine, lamivudine, abacavir, tenofovir disoproxil fumarate, emtricitabine, tenofovir alafenamide fumarate, saquinavir, ritonavir, indinavir, nelfinavir, amprenavir, lopinavir, atazanavir, fosamprenavir, tipranavir, darunavir, cobicistat, dolutegravir, raltegravir, enfuvirtide, maraviroc, elvitegravir, tenofovir alafenamide, tenofovir disoproxil, amantadine, rimantadine, oseltamivir, zanamivir, peramivir, baloxavir, laninamivir, aciclovir, valaciclovir, idoxuridine, vidarabine, penciclovir, famciclovir, trifluridine, cidofovir, foscarnet, clevudine, telbivudine, entecavir, adefovir, besifovir, peginterferon-α-2a, peginterferon-α-2b, ribavirin, boceprevir, dasabuvir, daclatasvir, asunaprevir, sofosbuvir, elbasvir, grazoprevir, glecaprevir, pibrentasvir, ombitasvir, paritaprevir, interferon alfa-2a, interferon alfa-2b and imiquimod.

5. The pharmaceutical composition according to claim 1 or claim 2, wherein the antidepressant is TCAs (Tricyclid antidepressants), SSRIs(Selective serotonin reuptake inhibitors), MAOI(Monoamine oxidase inhibitors), SNRIs(Serotonin-norepinephrine reuptake inhibitors)-based compounds, its derivatives, or a pharmaceutically acceptable salt thereof.

6. The pharmaceutical composition according to claim 5, wherein the antidepressant is one or more compound selected from the group consisting of amitriptyline, clomipramine, nortriptyline, desipramine, imipramine, amoxapine, maprotiline, trimipramine, protriptyline, melitracen, fluoxetine, fluvoxamine, paroxetine, sertraline, escitalopram, vortioxetine, moclobemide, duloxetine, venlafaxine, desbenlafaxine, milnacipran, bupropion, mirtazapine, trazodone and tianeptine.

7. The pharmaceutical composition according to claim 2, wherein the biguanide-based compound is selected from the group consisting of metformin, phenformin, buformin and biguanide.

8. The pharmaceutical composition according to claim 1, wherein a blending ratio of the first component including an antiviral agent; and the second component including an antidepressant is from 0.0000001 to 10 parts by weight of the first component: 1 part by weight of the second component.

9. The pharmaceutical composition according to claim 2, wherein a blending ratio of the first component including an antiviral agent; the second component including an antidepressant; and the third component including biguanide-based compound or a pharmaceutically acceptable salt thereof is from 0.0000001 to 10 parts by weight of the first component: 1 part by weight of the second component : from 0.01 to 100000 parts by weight of the third component.

10. The pharmaceutical composition according to claim 2, wherein the salt is selected from the group consisting of inorganic acids such as hydrochloric acid, nitric acid, phosphoric acid, sulfuric acid, hydrobromic acid, hydroiodic acid, nitrous acid or phosphorous acid, aliphatic mono and dicarboxylates, phenyl-substituted alkanoates, hydroxy alkanoates and alkandioates, aromatic acids, and aliphatic and aromatic sulfonic acids, pharmaceutically non-toxic salts such as sulfates, pyrosulfates, bisulfates, sulfites, bisulfites, nitrates, phosphates, monohydrogen phosphates, dihydrogen phosphates, metaphosphates, pyrophosphate chloride, bromides, iodides, fluorides, acetates, propionates, decanoates, caprylates, acrylates, formates, isobutyrates, caprates, heptanoates, propiolates, oxalates, malonates, succinates, suberates, sebacates, fumarates, maleates, butyne-1,4-dioates, hexane-1,6-dioates, benzoates, chlorobenzoates, methylbenzoates, dinitrobenzoates, hydroxybenzoates, methoxybenzoates, phthalates, terephthalates, benzenesulfonates, toluenesulfonates, chlorobenzenesulfonates, xylenesulfonates, phenylacetates, phenylpropionates, phenylbutyrates, citrates, lactates, hydroxybutyrates, glycolates, malates, tartrates, methanesulfonates, propanesulfonates, naphthalene-1-sulfonates, naphthalene-2-sulfonates or mandelates, metal salts such as sodium, potassium and calcium.

11. The pharmaceutical composition according to claim 1 or claim 2, wherein the cancer is selected from the group consisting of (A) breast cancers, including (1) ductal carcinoma, including ductal carcinoma in situ (DCIS) (comedocarcinoma, cribriform, papillary, micropapillary), invasive ductal carcinoma (IDC), ductal carcinoma, mucinous (colloidal) carcinoma, papillary carcinoma, metaplastic carcinoma and inflammatory carcinoma; (2) lobular carcinomas, including lobular carcinoma in situ (LCIS) and invasive lobular carcinoma; and (3) Paget's disease of the nipple; (B) cancers of the female reproductive system, including (1) cancers of the cervix, including cervical intraepithelial neoplasia (grade I), cervical intraepithelial neoplasia (grade II), cervical intraepithelial neoplasia (grade III) (squamous cell carcinoma in situ), keratinizing squamous cell carcinoma, nonkeratinizing squamous cell carcinoma, verrucous carcinoma, adenocarcinoma in situ, adenocarcinoma in situ, endometrial type carcinoma, endometrioid adenocarcinoma, clear cell adenocarcinoma, adenoepithelioma, adenoid cystic carcinoma, small cell carcinoma and undifferentiated carcinoma; (2) cancers of the uterine body, including endometrioid carcinoma, adenocarcinoma, adenoacanthoma (adenocarcinoma with squamous metaplasia), adenoepithelioma (mixed adenocarcinoma and squamous cell carcinoma), mucinous adenocarcinoma, serous adenocarcinoma, clear cell adenocarcinoma, squamous cell adenocarcinoma and undifferentiated adenocarcinoma; (3) cancers of the ovary, including serous cystadenoma, serous cystadenoma, mucinous cystadenoma, mucinous cystadenoma, endometrioid tumor, endometrioid adenocarcinoma, clear cell tumor, clear cell cystadenoma and unclassified tumors; (4) cancers of the vagina, including squamous cell carcinoma and adenocarcinoma; and (5) vulvar cancers, including vulvar intraepithelial neoplasia (grade I), vulvar intraepithelial neoplasia (grade II), vulvar intraepithelial neoplasia (grade III) (squamous cell carcinoma in situ); squamous cell carcinoma, verrucous carcinoma, Paget's disease of the vulva, adenocarcinoma (NOS); basal cell carcinoma (NOS) and Bartholin gland carcinoma; (C) cancers of the male reproductive system, including (1) cancer of the penis, including squamous cell carcinoma; (2) cancers of the prostate, including adenocarcinomas, sarcomas, and transitional cell carcinomas of the prostate; and (3) cancers of the testes, including seminoma tumor, non-seminoma tumor, teratomas, embryonic carcinomas, yolk sac tumor and choriocarcinoma; (D) cancers of the heart system, including sarcomas (angiosarcoma, fibrosarcoma, rhabdomyosarcoma, liposarcoma), myxoma, rhabdomyoma, fibroma, lipoma and teratoma; (E) cancers of the respiratory system, including squamous cell carcinoma of the larynx, primary pleural mesothelioma and squamous cell carcinoma of the pharynx; (F) cancers of the lung, including squamous cell carcinoma (epidermoid carcinoma), a variant of squamous cell carcinoma, spindle cell carcinoma, small cell carcinoma, carcinoma of other cells, carcinoma of the intermediate cell type, complex oat cell carcinoma, adenocarcinoma, acinar adenocarcinoma, papillary adenocarcinoma, bronchoalveolar carcinoma, mucin-producing solid carcinoma, giant cell carcinoma, giant cell carcinoma, clear cell carcinoma and sarcoma; (G) cancers of the gastrointestinal tract, including (1) cancers of the ampulla of vater, including primary adenocarcinoma, carcinoid tumor and lymphoma; (2) cancers of the anal canal, including adenocarcinoma, squamous cell carcinoma and melanoma; (3) cancers of the extrahepatic bile duct, including carcinoma in situ, adenocarcinoma, papillary adenocarcinoma, adenocarcinoma, intestinal type, mucinous adenocarcinoma, clear cell adenocarcinoma, signet ring cell carcinoma, adenoepithelioma, squamous cell carcinoma, small cell (oat cell) carcinoma, undifferentiated carcinoma, carcinoma (NOS), sarcoma and carcinoid tumor; (4) cancers of the colon and rectum, including adenocarcinoma in situ, adenocarcinoma, mucinous adenocarcinoma (colloidal type; > 50% mucinous carcinoma), signet ring cell carcinoma (greater than 50% of signet ring cells), squamous cell (epidermoid) carcinoma, adenoepithelioma, small cell (oat cell) carcinoma, undifferentiated carcinoma, carcinoma (NOS), sarcoma, lymphoma and carcinoid tumor; (5) cancers of the esophagus, including squamous cell carcinoma, adenocarcinoma, leiomyosarcoma and lymphoma; (6) cancers of the gallbladder, including adenocarcinoma, adenocarcinoma, bowel type, adenoepithelioma, carcinoma in situ, carcinoma (NOS), clear cell adenocarcinoma, mucinous adenocarcinoma, papillary adenocarcinoma, signet ring cell carcinoma, small cell (oat cell) carcinoma, squamous cell carcinoma and undifferentiated carcinoma; (7) cancers of the lips and oral cavity, including squamous cell carcinoma; (8) cancers of the liver, including liver cancer (hepatocellular carcinoma), cholangiocarcinoma, hepatoblastoma, hemangiosarcoma, hepatocellular adenoma and hemangioma; (9) cancers of the exocrine pancreas, including ductal cell carcinoma, giant cell carcinoma multiforme, giant cell carcinoma, osteoclastoid type, adenocarcinoma, adenoepithelioma, mucinous (colloidal) carcinoma, cystadenoma, acinar cell carcinoma, papillary carcinoma, small cell (oat cell) carcinoma, mixed cell type, carcinoma (NOS), undifferentiated carcinoma, endocrine cell tumor arising from islet cells of Langerhans and carcinoid tumor; (10) cancers of the salivary glands, including acinar (acinar) cell carcinoma, adenoid cystic carcinoma (cylindroma), adenocarcinoma, squamous cell carcinoma, carcinoma in pleomorphic adenoma (malignant mixed tumor), mucoepidermoid carcinoma (well-differentiated or low grade) and mucoepidermoid carcinoma (poorly differentiated or high grade); (11) cancers of the stomach, including adenocarcinoma, papillary adenocarcinoma, tubular adenocarcinoma, mucinous adenocarcinoma, signet ring cell carcinoma, adenoepithelioma, squamous cell carcinoma, small cell carcinoma, undifferentiated carcinoma, lymphoma, sarcoma and carcinoid tumor; and (12) cancers of the small intestine, including adenocarcinoma, lymphoma, carcinoid tumor, Kaposi's sarcoma, leiomyoma, hemangioma, lipoma, neurofibromatosis and fibroma; (H) cancers of the urinary system, including (1) cancers of the kidney, including renal cell carcinoma, carcinoma of the Bellini collecting duct, adenocarcinoma, papillary carcinoma, tubular carcinoma, granular cell tumor, clear cell carcinoma (adenocarcinoma of the kidney), sarcoma of the kidney and nephroblastoma; (2) cancers of the renal pelvis and ureter, including transitional cell carcinoma, papillary transitional cell carcinoma, squamous cell carcinoma and adenocarcinoma; (3) cancers of the urethra, including transitional cell carcinoma, squamous cell carcinoma and adenocarcinoma; and (4) cancers of the bladder, including carcinoma in situ, transitional urothelial cell carcinoma, papillary transitional cell carcinoma, squamous cell carcinoma, adenocarcinoma, and undifferentiated carcinoma; and(I) cancers of muscles, bones and soft tissues, including (1) cancers of the bone, including (a) osteogenesis: osteosarcoma; (b) chondrogenesis: chondrosarcoma and mesenchymal chondrosarcoma; (c) giant cell tumor, malignant; (d) Ewing's sarcoma; (e) vascular tumors: hemangioendothelioma, hemangiopericytoma and hemangiosarcoma; (f) connective tissue tumors: fibrosarcoma, liposarcoma, malignant mesenchymoma and undifferentiated sarcoma; and (g) other tumors: chordoma and adamantinoma of the long bones; (2) cancers of soft tissue, including alveolar soft part sarcoma, angiosarcoma, epithelioid sarcoma, extraosseous chondrosarcoma, fibrosarcoma, leiomyosarcoma, liposarcoma, malignant fibrous histiocytoma, malignant hemangiopericytoma, malignant mesenchymoma, malignant Schwannoma, rhabdomyosarcoma, synovial sarcoma and sarcoma (NOS); (3) cancers of the nervous system, including cancers of the skull (osteoma, hemangioma, granuloma, xanthoma, and osteitis deformans), cancers of the meninges (meningioma, meningiosarcoma, and gliomatosis), cancers of the brain (astrocytoma, meduloblastoma, glioma, ependymal glioma, germinoma (pineal tumor), glioblastoma multiforme, oligodendrocytoma, schwannoma, retinoblastoma, and congenital tumor), and cancers of the spinal cord (neurofibromatosis, meningioma, glioma, sarcoma); (4) hematologic malignancies, including myeloid leukemia (acute and chronic), acute lymphoblastic leukemia, chronic lymphocytic leukemia, myeloproliferative disease, multiple myeloma; myelodysplastic syndrome, Hodgkin's disease and non-Hodgkin's lymphoma (malignant lymphoma); (5) cancers of the endocrine system, including (a) cancers of the thyroid gland, including papillary carcinoma (including those of the follicular region), follicular carcinoma, medullary carcinoma and undifferentiated (anaplastic) carcinoma; and (b) neuroblastomas, including sympathicogonioma, sympathoblastoma, malignant ganglioneuroma, ganglioneuroblastoma and ganglioneuroma; (6) cancers of the skin, including squamous cell carcinoma, spindle cell squamous cell carcinoma, basal cell carcinoma, adenocarcinoma arising from sweat glands or sebaceous glands, and malignant melanoma; and (7) cancers of the eye, including (a) cancer of the conjunctiva, including carcinoma of the conjunctiva; (b) cancers of the eyelid, including basal cell carcinoma, squamous cell carcinoma, melanoma of the eyelid and sebaceous cell carcinoma; (c) cancers of the lacrimal gland, including adenocarcinoma, adenoid cystic carcinoma, carcinoma in pleomorphic adenoma, mucoepidermoid carcinoma and squamous cell carcinoma; (d) cancers of the uvea, including spindle cell melanoma, mixed cell melanoma and epithelioid cell melanoma; (e) cancers of the orbit, including sarcoma of the orbit, soft tissue tumor, and sarcoma of the bone; and (f) retinoblastoma.

12. The pharmaceutical composition according to claim 1 or claim 2, wherein the first component and the second component are formulated into a formulation selected from the group consisting of a tablet, a capsule, an injection, a troche, a powder, a granule, a solution, a suspension, an oral solution, an emulsion, a syrup, a suppository, a vaginal tablet and a pill.

13. A complex, mixed or combined kit for the prevention or treatment of cancer, comprising a preparation containing an antiviral agent and a preparation containing an antidepressant agent as active ingredients.

14. A complex, mixed or combined kit for the prevention or treatment of cancer, comprising a preparation containing an antiviral agent, a preparation containing an antidepressant agent, and a preparation containing a biguanide-based compound or a pharmaceutically acceptable salt thereof as active ingredients.

15. The kit according to claim 13 or claim 14, wherein the complex, mixed or combined kit are formulated into a formulation selected from the group consisting of a tablet, a capsule, an injection, a troche, a powder, a granule, a solution, a suspension, an oral solution, an emulsion, a syrup, a suppository, a vaginal tablet and a pill.

16. A method for treating cancer, comprising the step of administering a complex, mixture, or combination of a pharmaceutically effective amount of a first component including an antiviral agent and a second component including an antidepressant to a subject with cancer.

17. The method for treating cancer, wherein further administering a biguanide-based compound or a pharmaceutically acceptable salt thereof.

18. A complex, mixed or combined formulation for the prevention or treatment of cancer, comprising a first component including an antiviral agent and a second component including an antidepressant as active ingredients.

19. The formulation according to claim 18, wherein further comprising a biguanide-based compound or a pharmaceutically acceptable salt thereof.
